# EUROPEAN PATENT SPECIFICATION

(11) **EP 1 218 064 B1**
(45) Date of publication and mention of the grant of the patent: **01.06.2005**
(21) Application number: 00961433.0
(22) Date of filing: 31.08.2000
(51) Int. Cl.: A61P 43/00, A61K 45/06

(54) **COMPOSITIONS FOR TREATING AUTOIMMUNE DISEASEs CONTAINING A COMPOUND WHICH INHIBITS ICAM-LFA-1 INTERACTION AND A COMPOUND WHICH INHIBITS CD40-CD40 LIGAND INTERACTION**
ZUSAMMENSETZUNGEN ZUR BEHANDLUNG VON AUTOIMMUNKRANKHEITEN, DIE MITTEL, WELCHE ICAM-1/LFA-1 INTERAKTIONEN INHIBIEREN UND MITTEL, WELCHE CD40-CD40-LIGANDE INTERAKTIONEN INHIBIEREN, ENTHALTEN
COMPOSITIONS POUR TRAITER LES MALADIES AUTOIMMUNES CONTENANT UN COMPOSE INHIBANT L'INTERACTION ICAM-LFA-1 ET UN COMPOSE INHIBANT L'INTERACTION ENTRE CD40 ET SON LIGAND

(30) Priority: 01.09.1999 US 151953 P; 02.09.1999 US 152168 P; 12.11.1999 US 164854 P
(43) Date of publication of application: 03.07.2002
(73) Proprietor: BOEHRINGER INGELHEIM PHARMACEUTICALS INC., Ridgefield, Connecticut 06877-0368 (US)
(72) Inventor: SOUZA, Donald, J., Berlin, CT 06037 (US)
(74) Representative: Chapman, Paul William
(86) International application number: PCT/US2000/023873
(87) International publication number: WO 2001/015733

(56) References cited:
- WO-A-90/03400
- HARRISON, PAUL C. (1) ET AL: "Cyclosporine dosage can be reduced when used in combination with an anti-intercellular adhesion molecule-1 monoclonal antibody in rats undergoing heterotopic heart transplantation." JOURNAL OF HEART AND LUNG TRANSPLANTATION, (FEB., 1998) VOL. 17, NO. 2, PP. 150-157. , XP000989668
- SOUZA, D. (1) ET AL: "Synergistic inhibition of established collagen induced arthritis (CIA) through dual inhibition of ICAM -1 and CD40L pathways." ARTHRITIS & RHEUMATISM, (SEPT., 1999) VOL. 42, NO. 9 SUPPL., PP. S124. MEETING INFO.: 63RD ANNUAL SCIENTIFIC MEETING OF THE AMERICAN COLLEGE OF RHEUMATOLOGY AND THE 34TH ANNUAL SCIENTIFIC MEETING OF THE ASSOCIATION OF RHEUMATOLOGY HEALTH PROFESSIONAL, XP000987215
- HARRISON, PAUL C. (1) ET AL: "Anti-LFA-1alpha reduces the dose of cyclosporin A needed to produce immunosuppression in heterotopic cardiac transplanted rats." JOURNAL OF HEART AND LUNG TRANSPLANTATION, (APRIL, 1999) VOL. 18, NO. 4, PP. 279-284. , XP000989586

## Description

### Background of the Invention

### Field of the Invention

The present invention relates generally to the treatment of autoimmune disease, and to compositions useful in treating autoimmune disease.

### Related Art

Autoimmune diseases occur when immune reactivity to antigen arises through a breakdown in the mechanisms which control tolerance. Rheumatoid arthritis and systemic lupus erythematosus (SLE) are two examples of autoimmune disease. Rheumatoid arthritis is a clinical syndrome of unknown cause characterized by symmetric, polyarticular inflammation of synovial-lined joints. This inflammation commonly involves extraarticular tissues, including pericardium lung, and blood vessels (Harris, E.D., *New Engl. J. Med. 322:* 1277-1289 (1990)).

Systemic lupus erythematosus (SLE) is an inflammatory disease characterized by formation of antinuclear antibodies and deposition of immune complexes, and is manifested by inflammatory skin lesions, mucositis, serositis, vasculitis, and glomerulonephritis (Mills, J.A., *New Engl. J. Med. 330*: 1871-1879 (1994)). SLE is a chronic inflammatory autoimmune disorder involving the joints, skin, kidney, and other organs. Lupus nephritis or lupus glomerulonephritis is the renal component commonly observed in SLE patients. The production of autoantibodies, deposition of immune complexes, and activation of the complement system are all early events leading to nephritis in lupus patients. The infiltration of inflammatory cells and their interaction with resident renal cells is involved in the progression of renal injury and the amplification of inflammatory responses in lupus nephritis.

Cellular adhesion molecules may be involved in the pathogenesis of rheumatoid arthritis (Cronstein, B.N., *Curr. Opinion Rheum. 6*: 300-304 (1994)). ICAM-1 is a cellular adhesion molecule, and is an important cell-surface glycoprotein regulating interactions among immune cells and between accessory cells and T-lymphocytes (Wuthrich, R.P. *et al., Am. J. Pathol. 136*: 441-450 (1990)). ICAM-1 binds to lymnphocyte function-associated antigen-1 (LFA-1) (Kakimoto, K. *et al., Cellular Immunol. 142:* 326-337 (1992)).

ICAM-1/LFA-1 interactions play a major role in T lymphocyte activation by antigen-presenting cells, and mediate leukocyte homotypic and heterotypic adhesion functions (Davis *et al., Structure, Function, and Regulation of Molecules in Leukocyte Adhesion,* Lipsky *et al*., Eds., Springer-Verlag, New York, p. 256 (1993); Kuhlman *et al., J. Immunol. 146:* 1773 (1991).

The binding of the CD40 ligand (also known as gp39, CD 154, CD40L, TRAP, or TBAM) molecule on the T helper cell surface to B cell surface CD40 is the molecular event that mediates direct T cell help for B cell activation (Koshy, M. *et al., J. Clin. Invest.* 98: 826-837 (1996)). CD40/CD40L interaction is critical for the production of antibodies against T-dependent antigens, germinal center formation, B cell proliferation and differentiation, isotope switching, and generation of B cell memory (Durie *et al., Immunol. Today 15*: 406 (1994)). CD40 is a member of the TNF receptor family of molecules and is expressed on a variety of cell types, including B cells and other antigen presenting cells, endothelial cells, and keratinocytes. (Grewal *et al., Ann. Rev. Immunol. 16:* 111-135 (1998)).

ICAM-1 and CD40 may be involved in the pathogenesis of rheumatoid arthritis (Koshy, M. *et al., J. Clin. Invest. 98:* 826-837 (1996); Wuthrich, R.P. *et al., Am. J. Pathol. 136:* 441-450 (1990)). CD40 may be involved in the pathogenesis of systemic lupus erythematosus (Kalled, S.L. *et al, J. Immutiology* *160:* 2158 (1990)). Therapeutic effects of anti-CD40 ligand or anti-adhesion molecule treatments on disease severity in systemic lupus erythematosus patients are not known (McMurray, R.W., *Semin. Arthritis Rheum. 25:* 2151-234 (1996)).

Prior to this invention, therapeutic remedies for autoimmune diseases, and particularly rheumatoid arthritis and SLE, remain elusive. Accordingly, there is a need in the art for therapeutic compositions and methods for effectively treating auto-immune diseases, and particularly rheumatoid arthritis and SLE.

### Summary of the Invention

The present invention provides a pharmaceutical composition for treating auto-immune disease, the composition comprising a pharmaceutically acceptable carrier and a synergistic ratio of (i) an agent that interrupts or perturbs ICAM-LFA-1 interaction, and an agent that interrupts or perturbs CD40-CD40 ligand interaction.

The present invention also provides the use of such pharmaceutical compositions in the manufacture of a medicament.

The present invention provides the use of a synergistic ratio of (i) an agent that interrupts or perturbs ICAM-LFA-1 interaction, and (ii) an agent that interrupts or perturbs CD40-CD40 ligand interaction in the manufacture of a medicament for treating auto-immune disease.

The present invention also provides the use of a synergistic ratio of (i) an antibody that interrupts or perturbs ICAM-LFA-1 interaction, or an active fragment thereof, and (ii) an that interrupts or perturbs CD40-CD40 ligand interaction, or an active fragment thereof in the manufacture of a medicament for treating rheumatoid arthritis and systemic lupus erythematosus, particuarly end-stage systemic lupus erythematosus.

It has been found that coadministration of an agent that interrupts or perturbs ICAM-LFA-1 interaction and an agent that interrupts or perturbs CD40-CD40 ligand interaction provides a synergistic therapeutic effect. The present invention provides compositions and medicaments for treating autoimmune disease, particularly rheumatoid arthritis and systemic lupus erythematosus. Using the compositions and medicaments of the present invention, autoimmune diseases, and particularly rheumatoid arthritis and SLE, can be treated.

### Brief Description of the Figures

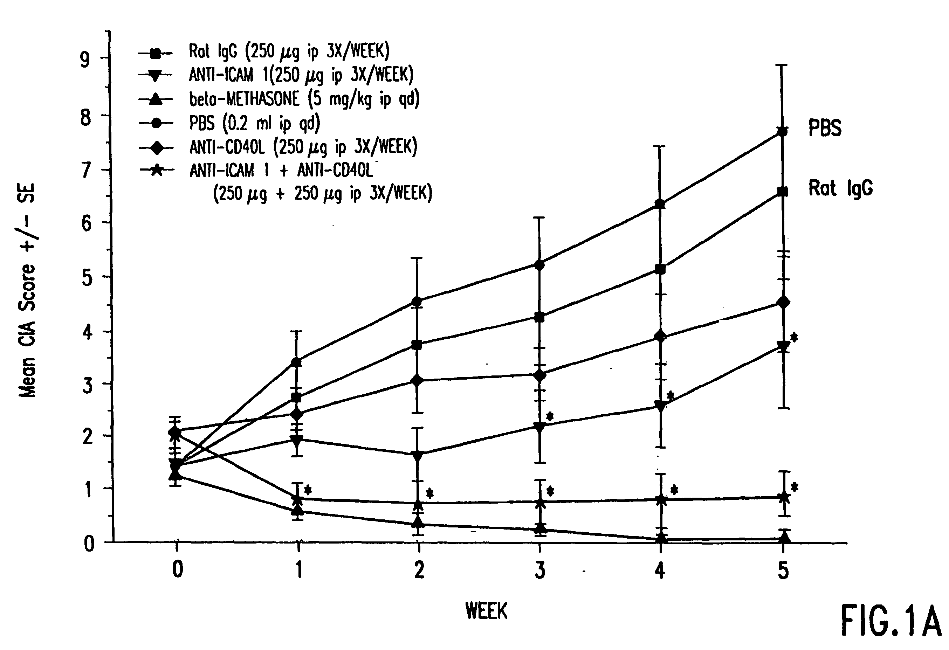

FIG. 1A depicts steroid-like inhibition of established collagen-induced arthritis (CIA) through anti-CD40 ligand antibody and anti-ICAM-1 antibody combination therapy. The mean arthritis severity score is shown (± standard error) over time for each respective treatment. (■): rat IgG (250 µg i.p. 3 times/week); (▼): YN1/1 airti-ICAM-1 antibody (250 pg i.p. 3 times/week); (▲): beta-methasone (5 mg/kg/i.p. every day); (●): phosphate-buffered saline (0.2 ml i.p. everyday); (◆): MR-1 anti-CD40 ligand antibody (250 µg i.p. 3 times/week); ( 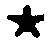 ); YN1/1 anti-ICAM-1 antibody (250 µg i.p. 3 times/week)/ MR-1 anti-CD40 ligand antibody (250 µg i.p. 3 times/week). * p < 0.05 vs. rat IgG and PBS controls.

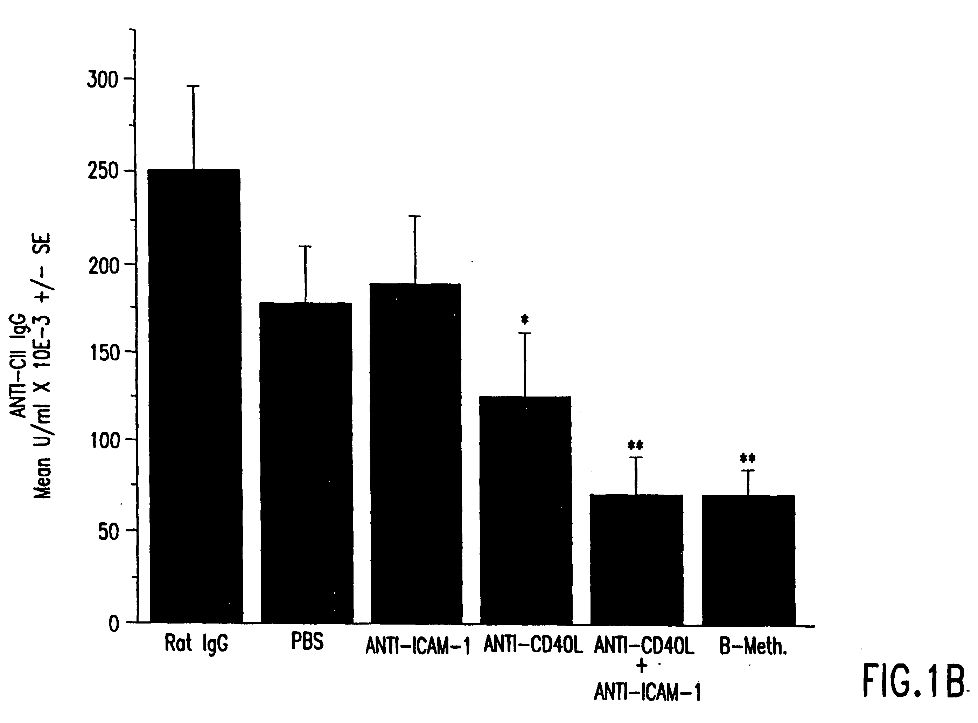

FIG. 1B is a bar graph depicting the level of type II collagen IgG 5 weeks after the indicated treatments. Anti-CII IgG was assayed in an ELISA assay, and is shown as the mean units peroxidase activity (OD=492 nm) per ml X 10⁻³ ± standard error. * p < 0.05 vs. rat IgG control. ** p < 0.05 vs. rat IgG and phosphate-buffered saline (PBS) controls.

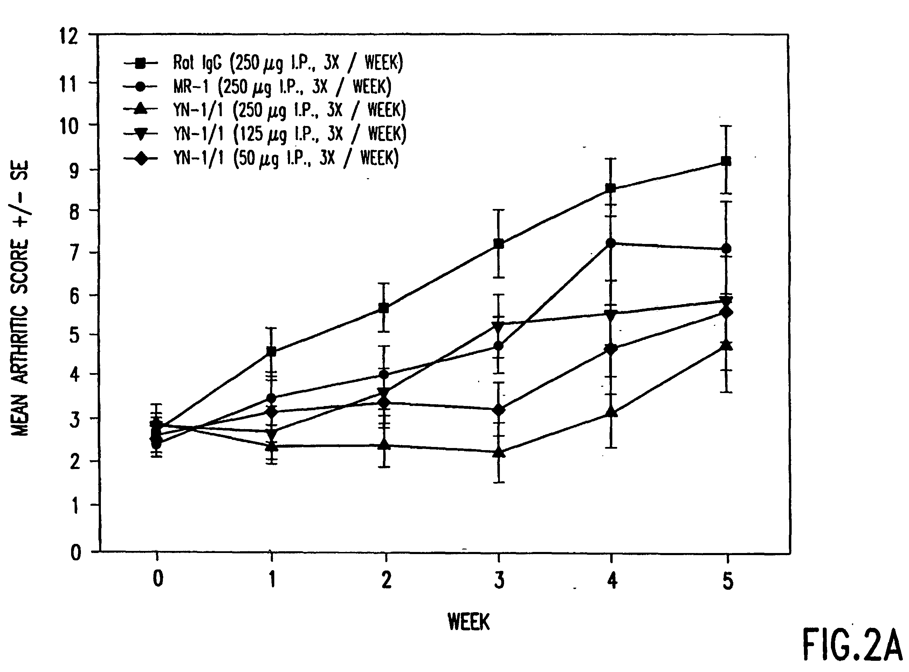

FIG. 2A depicts the effect of anti-CD40L antibody and of anti-ICAM-1 antibody dosage on the mean arthritic severity score. The mean arthritis severity score is shown (± standard error) over time for each respective treatment. (■): rat IgG (250 µg i.p. 3 times/week); (●): MR-1 anti-CD40 ligand antibody (250 µg i.p. 3 times/week); (▲): YN1/1 anti-ICAM-1 antibody (250 µg i.p. 3 times/week); (▼): YN1/1 anti-ICAM-1 antibody (125 µg i.p. 3 times/week); (◆): YN1/1 anti-ICAM-1 antibody (50 µg i.p. 3 times/week).

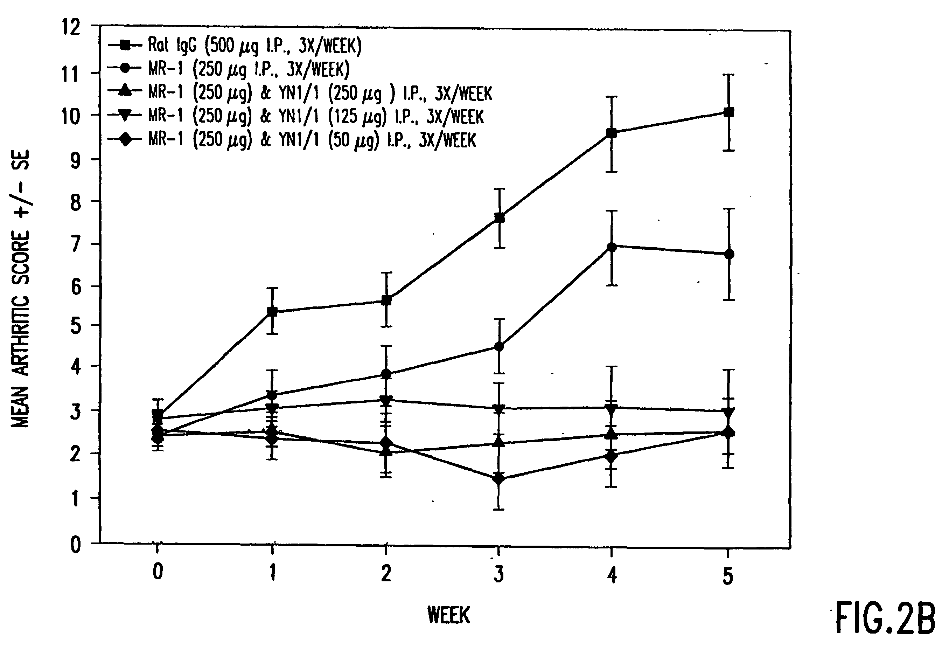

FIG. 2B depicts the synergistic effect of anti-CD40L antibody and ofanti-ICAM-1 antibody dosage on the mean arthritic severity score for anti-CD40L and anti-ICAM-1 combination therapy. The mean arthritis severity score is shown (± standard error) over time for each respective treatment. (■): rat IgG (500 µg i.p. 3 times/week); (●): MR-1 anti-CD40 ligand antibody (250 µg i.p. 3 times/week); (▲): MR-1 anti-CD40 ligand antibody (250 µg i.p. 3 times/week) and YN1/1 anti-ICAM-1 antibody (250 µg i.p. 3 times/week); (▼): MR-1 anti-CD40 ligand antibody (250 µg i.p. 3 times/week) and YN1/1 anti-ICAM-1 antibody (125 µg i.p. 3 times/week); (◆): MR-1 anti-CD40 ligand antibody (250 µg i.p. 3 times/week) and YN1/1 anti-ICAM-1 antibody (50 µg i.p. 3 times/week).

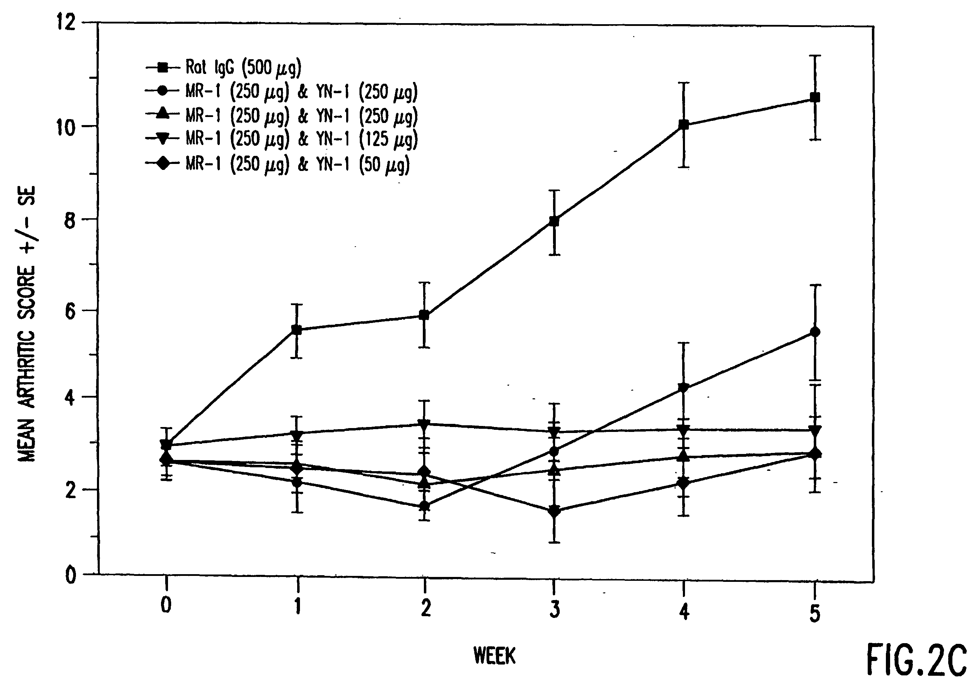

FIG. 2C depicts the synergistic effect of anti-CD40L antibody and of anti-ICAM-1 antibody dosage on the mean arthritic severity score for anti-CD40L and anti-ICAM-1 combination therapy. The mean arthritis severity score is shown (± standard error) over time for each respective treatment. (■): rat IgG (500 µg i.p. Q.I.D., MWF for 5 weeks); (●): MR-1 anti-CD40 ligand antibody (250 µg i.p. Q.I.D., MWF for 5 doses); (▲): MR-1 anti-CD40 ligand antibody (250 pg i.p. Q.I.D., MWF for 5 weeks) and YN1/1 anti-ICAM-1 antibody (250 µg i.p. Q.I.D., MWF for 5 weeks); (▼): MR-1 anti-CD40 ligand antibody (250 µg i.p. Q.I.D., MWF for 5 weeks) and YN1/1 anti-ICAM-1 antibody (125 µg i.p. Q.I.D., MWF for 5 weeks); (◆): MR-1 anti-CD40 ligand antibody (250 µg i.p. Q.I.D., NFWF for 5 weeks) and YN1/1 anti-ICAM-1 antibody (50 µg i.p. Q.I.D., MWF for 5 weeks).

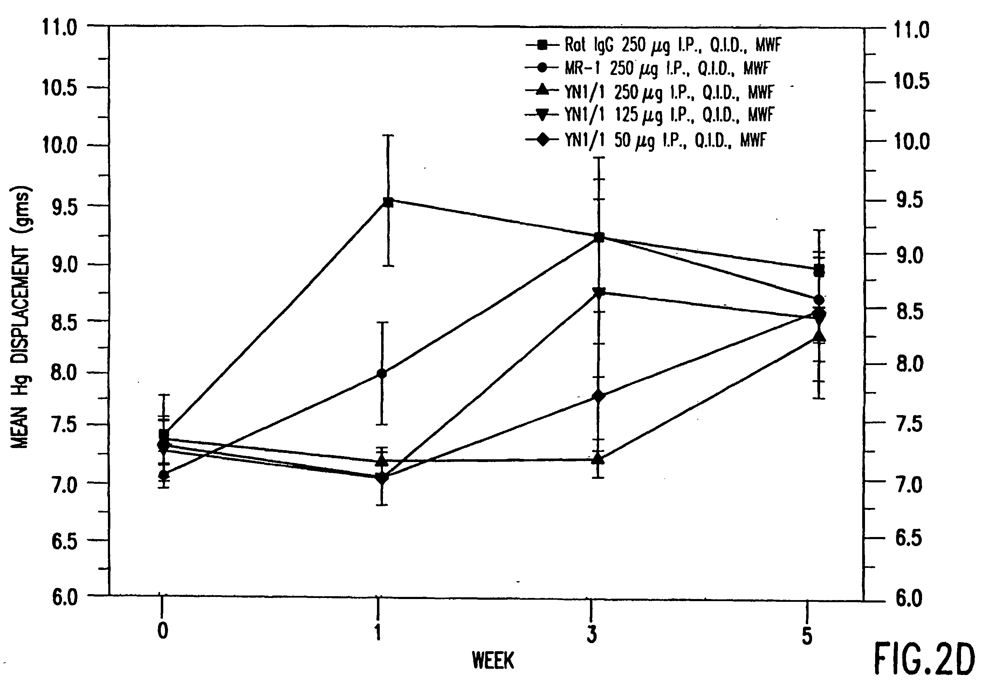

FIG. 2D depicts the effect of anti-ICAM-1 antibody or anti-CD40L antibody on mean mercury (Hg) displacement in an assay of inflammation based on paw volume. (■): rat IgG (250 µg i.p. Q.I.D., MWF); (●): MR-1 anti-CD40 ligand antibody (250 µg i.p. Q.I.D., MWF); (▲): YN1/1 anti-ICAM1 antibody (250 µg i.p., Q.I.D., MWF); (▼): YN1/1 anti-ICAM1 antibody (125 µg i.p., Q.I.D., MWF); (◆): YN1/1 anti-ICAM1 antibody (50 µg i.p., Q.I.D., MWF).

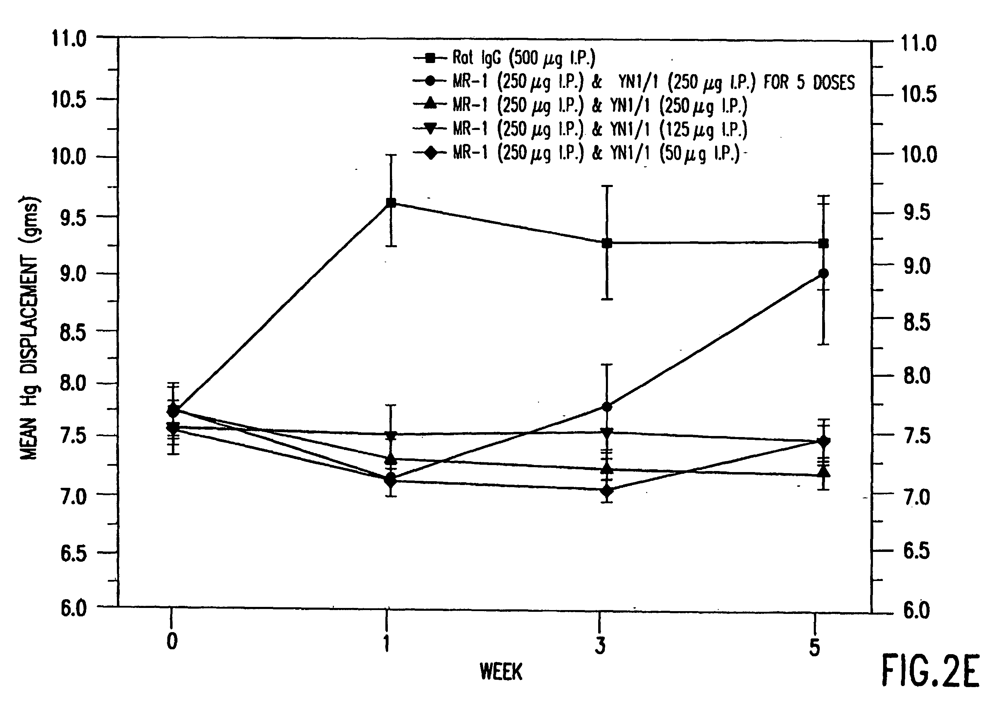

FIG. 2E depicts the synergistic effect of anti-ICAM-1 antibody and anti-CD40L antibody on mean mercury (Hg) displacement in an assay of inflammation based on paw volume. (■): rat IgG (500 µg i.p.); (●): MR-1 anti-CD40 ligand antibody (250 µg i.p.) and YN1/1 anti-ICAM1 antibody (250 µg i.p.) for 5 doses; (▲): MR-1 anti-CD40 ligand antibody (250 µg i.p.) and YN1/1 anti-ICAM1 antibody (250 µg i.p.) for five weeks; (▼): MR-1 anti-CD40 ligand antibody (250 µg i.p.) and YN1/1 anti-ICAM1 antibody (125 µg i.p.) for five weeks; (◆): MR-1 anti-CD40 ligand antibody (250 µg i.p.) and YN1/1 anti-ICAM1 antibody (50 µg i.p.) for five weeks.

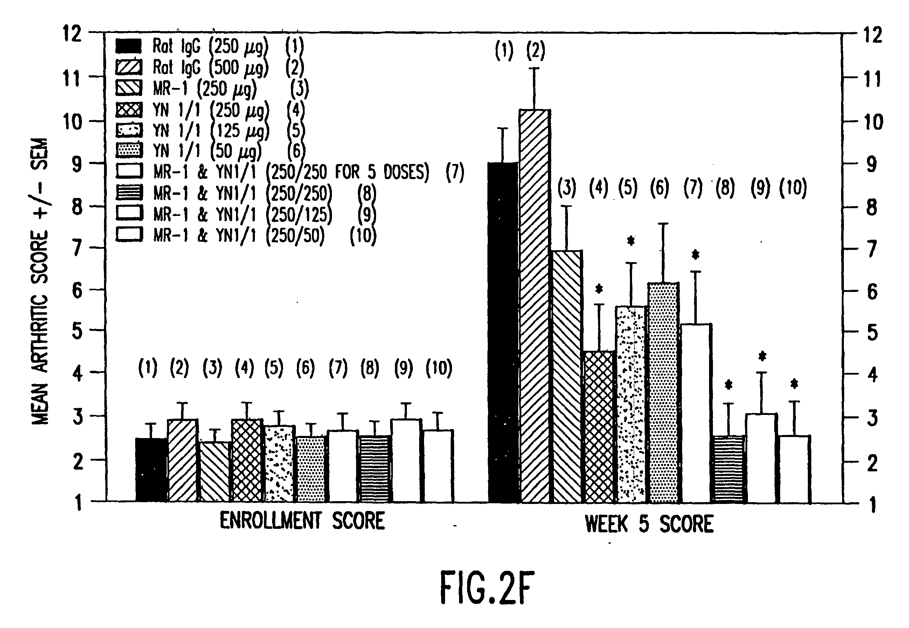

FIG. 2F is a bar graph depicting the synergistic inhibition of established arthritis through anti-ICAM-1 and anti-CD40L combination therapy. The mean arthritic score is shown at enrollment and week 5 (+ standard error) for each respective treatment. * p < 0.05 vs. rat IgG controls. Integers in parentheses correlate the respective treatments listed in the figure legend with the respective mean arthritic scores in the bar graph.

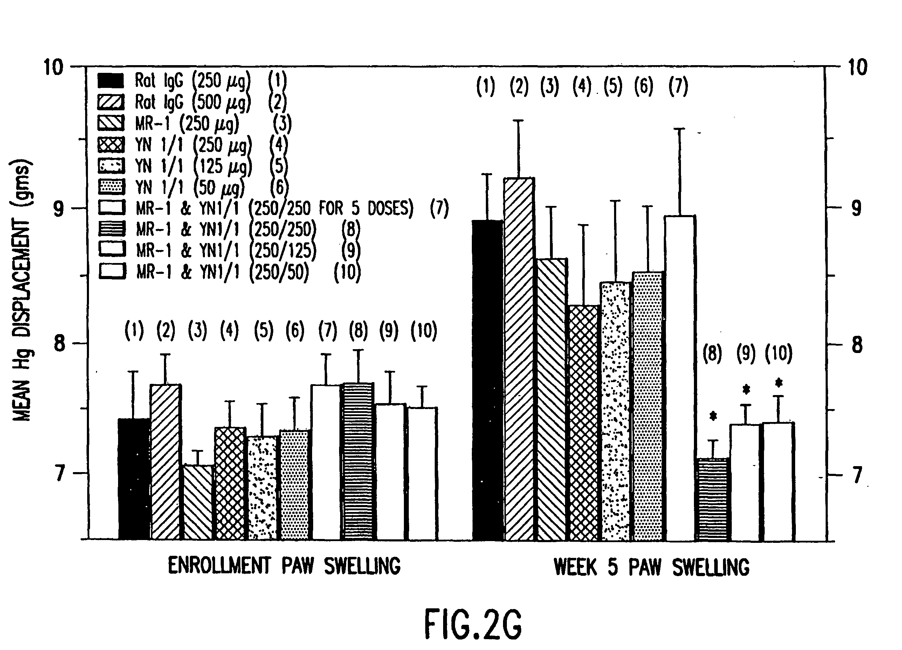

FIG. 2G is a bar graph depicting the synergistic inhibition of established edema through anti-ICAM-1 and anti-CD40L combination therapy. The mean Hg displacement is shown at enrollment and week 5 (+ standard error) for each respective treatment. * p < 0.05 vs. rat IgG controls. Integers in parentheses correlate the respective treatments listed in the figure legend with the respective mean arthritic scores in the bar graph.

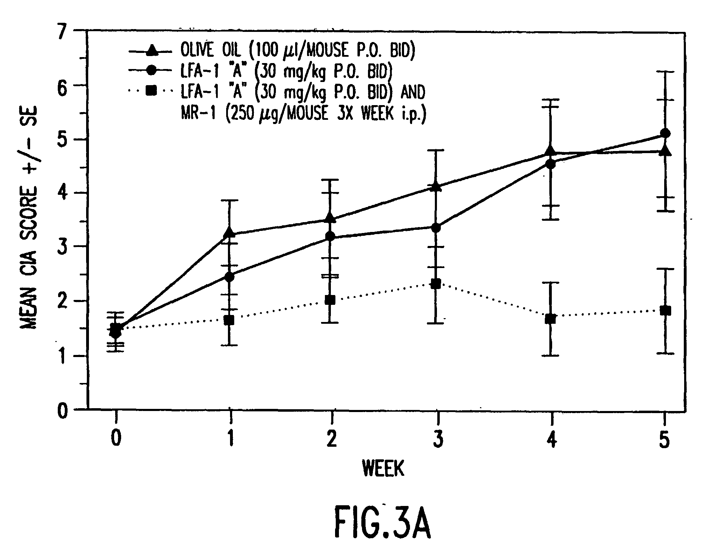

FIG. 3 A depicts the effect of anti-CD40L antibody and LFA-1 antagonist combination therapy on the mean arthritic severity score. The mean arthritis severity score is shown (± standard error) over time for each respective treatment. (▲): olive oil control (100 µl P.O. b.i.d.); (●): LFA-1 antagonist "A" (LFA-1 "A") (30 mg/kg P.O. b.i.d.); (■): LFA-1 antagonist "A" (30 mg/kg P.O. b.i.d.) and MR-1 anti-CD40L antibody (250 µg i.p. 3X/week).

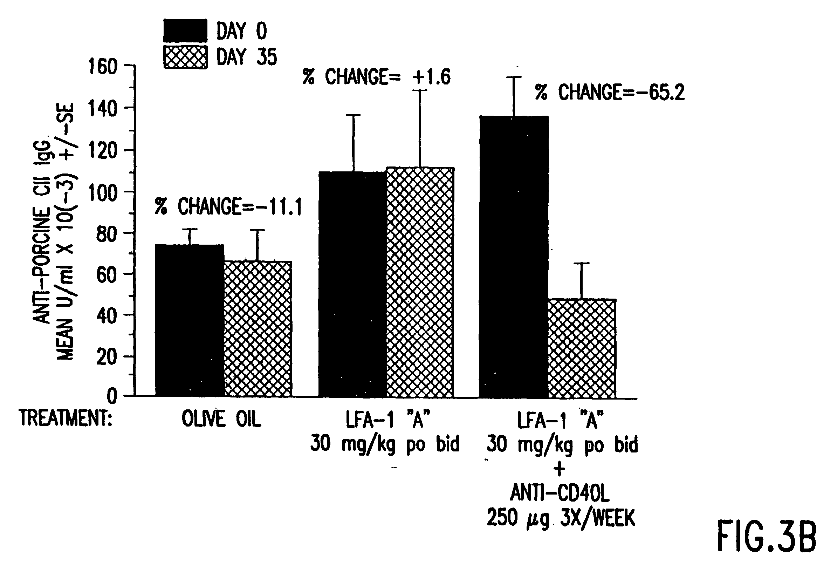

FIG 3B. is a bar-graph depicting the level of type II collagen IgG 5 weeks after the indicated treatments. Anti-CII IgG was assayed in an ELISA assay, and is shown as the mean units peroxidase activity (OD=492 nm) per ml X 10⁻³ ± standard error.

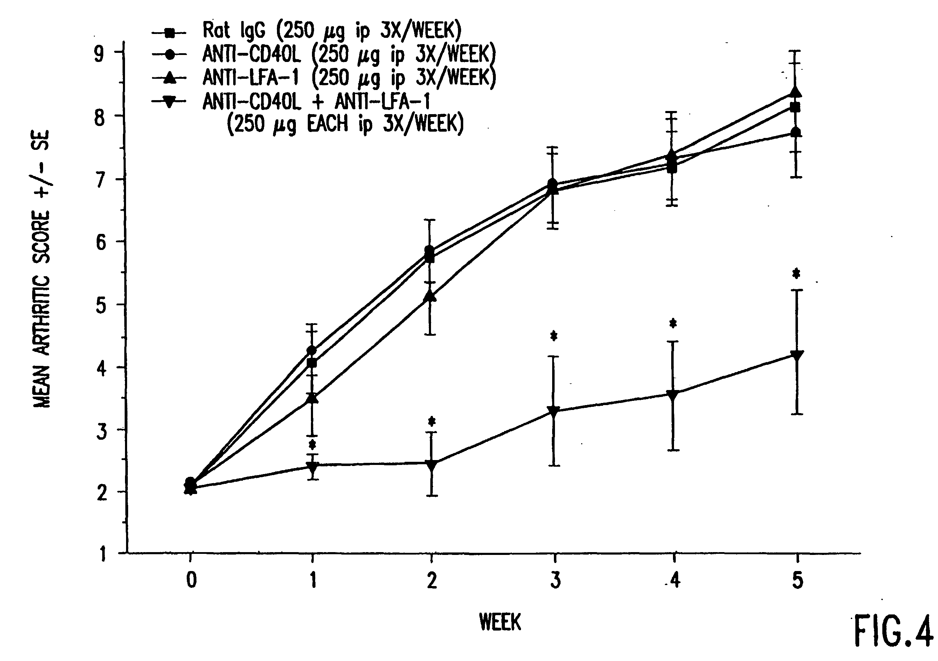

FIG. 4 depicts the synergistic inhibition of established arthritis through anti-LFA-1 and anti-CD40L combination therapy. The mean arthritic score is shown (+ standard·error) for each respective treatment. (▲): mAb M17/4.4 rat anti-mouse LFA-1 (250 µg i.p. 3X/week); (●): mAb MR-1 hamster anti-CD40 ligand antibody (250 µg i.p. 3X/week); (▼): combination M 17/4.4 + MR-1 (250 µg + 250 µg i.p. 3X/week). Control mice received: (■): rat IgG (250 µg i.p. 3X/week); * p < 0.05 vs. rat IgG controls.

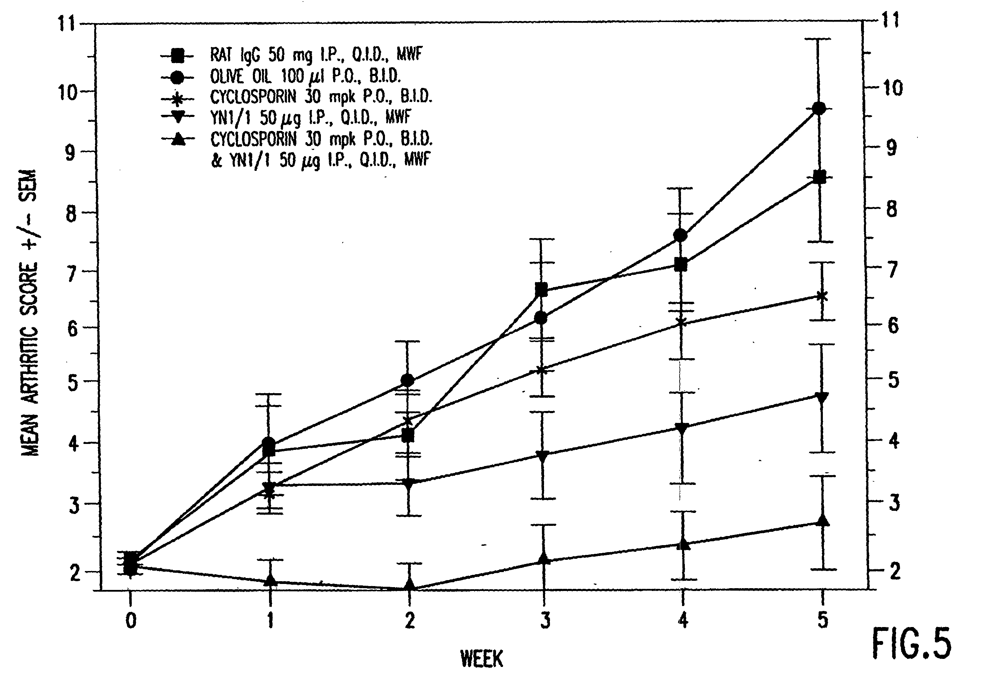

FIG. 5 depicts the effect of anti-ICAM-1 antibody and cyclosporin on the mean arthritic severity score. The mean arthritis severity score is shown (± standard error) over time for each respective treatment. (■): rat IgG (50 µg i.p., Q.I.D., MWF); (●): olive oil (100 µl P.O., B.I.D.); (*): cyclosporin 30 mg/kg P.O., B.I.D.); (▼): YN1/1 anti-ICAM-1 antibody (50 µg i.p., Q.I.D., MWF); (▲): cyclosporin 30 mg/kg P.O., B.I.D.) and YN1/1 anti-ICAM-1 antibody (50 µg i.p., Q.I.D., MWF).

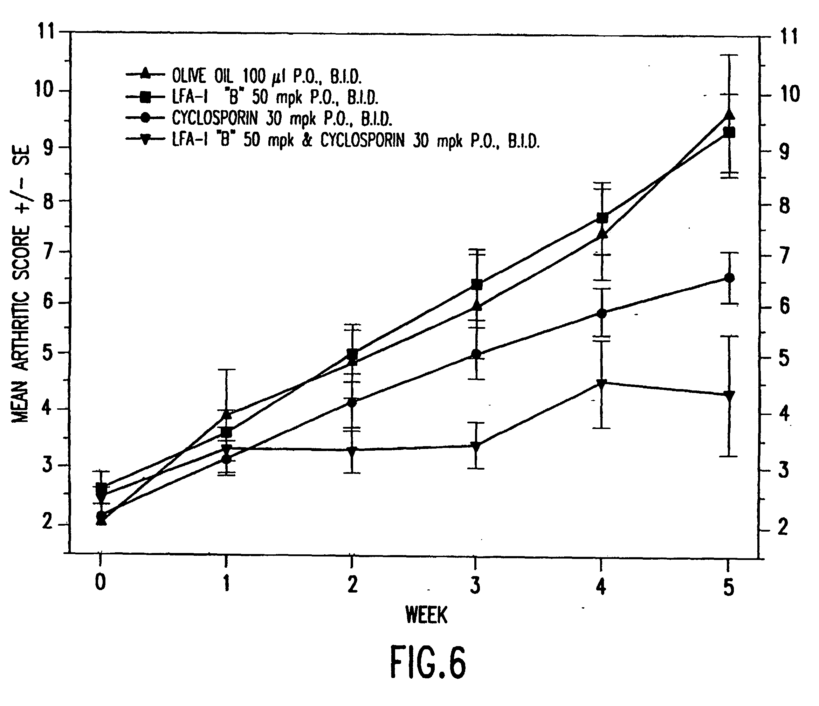

FIG. 6 depicts the effect of LFA-1 antagonist and non-cytotoxic immunosuppressive combination therapy on the mean arthritic severity score. The mean arthritis severity score is shown (± standard error) over time for each respective treatment. (▲): olive oil control (100 µl P.O. b.i.d.); (■): LFA-1 antagonist "B" (LFA-1 "B") (50 mg/kg P.O. b.i.d.); (●): cyclosporin (30 mg/kg P.O. b.i.d.); (▼): LFA-1 antagonist "B" (50 mg/kg P.O. b.i.d.) and cyclosporin (30 mg/kg P.O. b.i.d.).

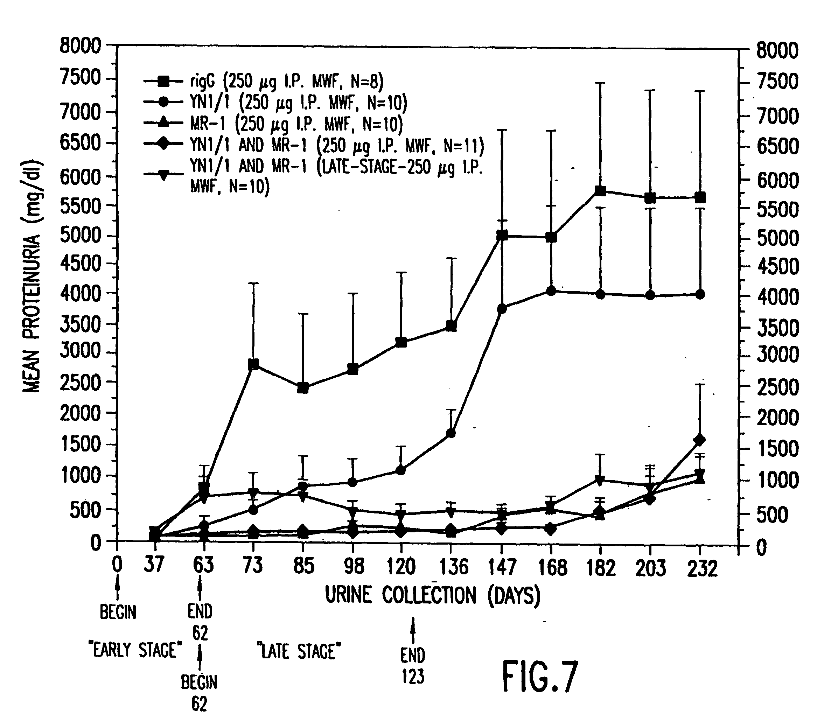

FIG. 7 depicts the effect of various treatments on the mean proteinuria level in (NZB/NZW)F₁ SLE mice. (■): rat IgG (250 µg i.p. MWF, N=8); (●): YN1/1 anti-ICAM-1 antibody (250 µg i.p. MWF, N=10); (▲): MR-1 anti-CD40 ligand antibody (250 µg i.p. MWF, N=10); (◆): YN1/1 anti-ICAM-1 antibody and MR-1 anti-CD40 ligand antibody (each antibody administered at 250 µg i.p. MWF, N=11); (▼): late stage: YN1/1 anti-ICAM-1 antibody and MR-1. anti-CD40 ligand antibody (each antibody administered at 250 µg i.p. MWF, N=10).

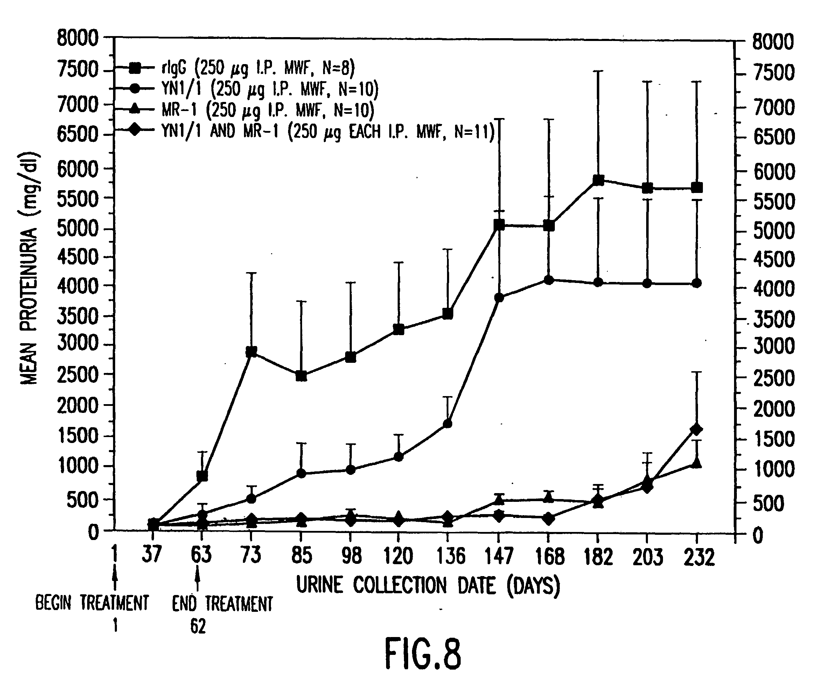

FIG. 8 depicts the effect of various treatments on the mean proteinuria level in (NZB/NZW)F₁ SLE mice in early stage disease. (■): rat IgG (250 µg i.p. MWF, N=8); (●): YN1/1 anti-ICAM-1 antibody (250 pg i.p. MWF, N=10); (▲): MR-1 anti-CD40 ligand antibody (250 µg i.p. MWF, N=10); (◆): YN1/1 anti-ICAM-1 antibody and MR-1 anti-CD40 ligand antibody (each antibody administered at 250 µg i.p. MWF, N=11). Treatments were ended two months after they were begun.

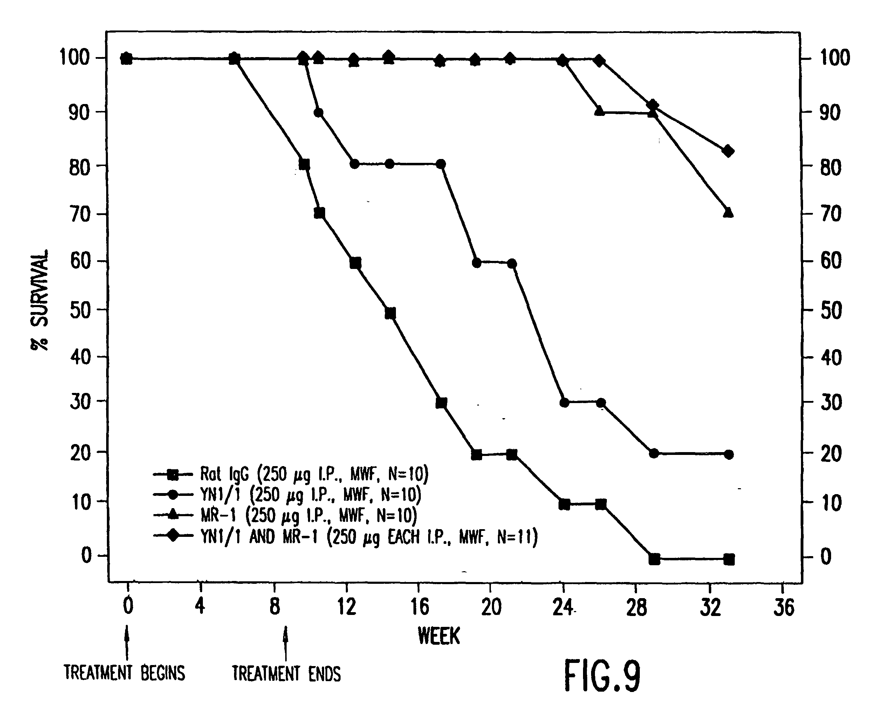

FIG. 9 depicts the effect of various treatments on the % survival of (NZB/NZW)F₁ SLE mice in early stage disease. (■): rat IgG (250 µg i.p. MWF, N=10); (●): YN1/1 anti-ICAM-1 antibody (250 µg i.p. MWF, N=10); (▲): MR-1 anti-CD40 ligand antibody (250 µg i.p. MWF, N=10); (◆): YN1/1 anti-ICAM-1 antibody and MR-1 anti-CD40 ligand antibody (each antibody administered at 250 µg i.p. MWF, N=11). Treatments were ended two months after they were begun.

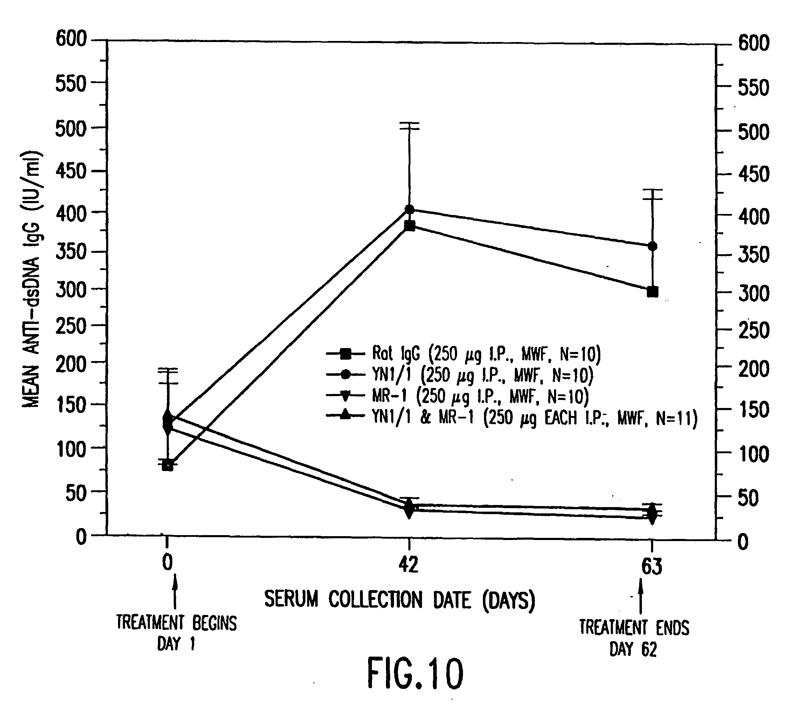

FIG. 10 depicts the effect ofvarious antibody treatments on the mean level of anti-dsDNA IgG in (NZB/NZW)F₁ SLE mice in the early stage disease. (■): rat IgG (250 µg i.p. MWF, N=10); (●): YN1/1 anti-ICAM-1 antibody (250 µg i.p. MWF, N=10); (▼): MR-1 anti-CD40 ligand antibody (250 µg i.p. MWF, N=10); (▲): YN1/1 anti-ICAM-1 antibody and MR-1 anti-CD40 ligand antibody (each antibody administered at 250 µg i.p. MWF, N=11).

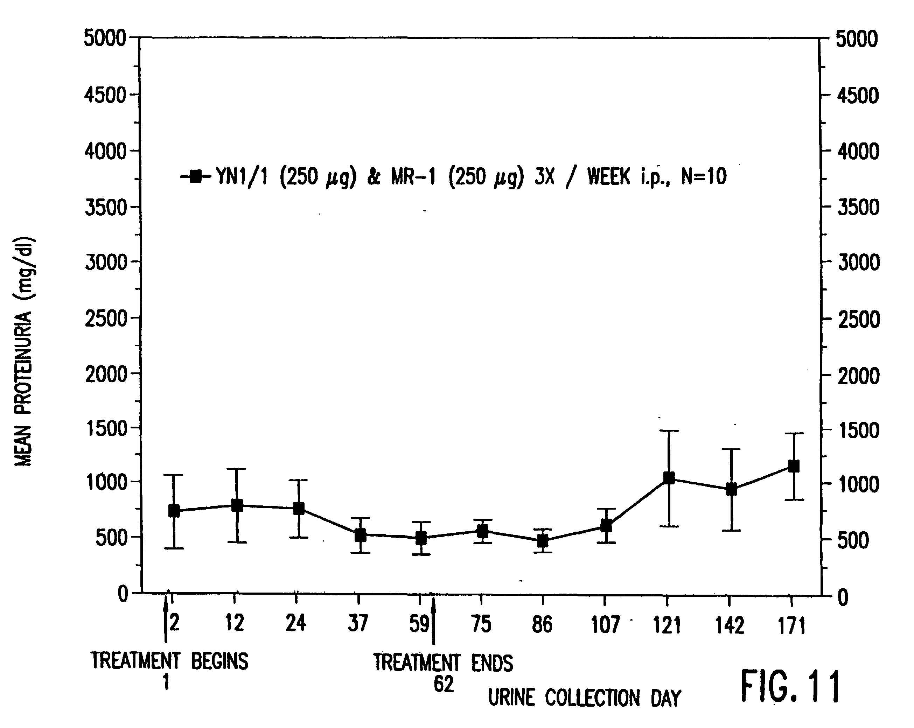

FIG. 11 depicts the effect of YN1/1 anti-ICAM-1 antibody and MR-1 anti-CD40L antibody on the mean proteinuria level in (NZB/NZW)F₁ SLE mice in late stage disease. (■): YN1/1 anti-ICAM-1 antibody and MR-1 anti-CD40 ligand antibody (each antibody administered at 250 µg i.p. 3 times/week, N=10). Treatments were ended two months after they were begun.

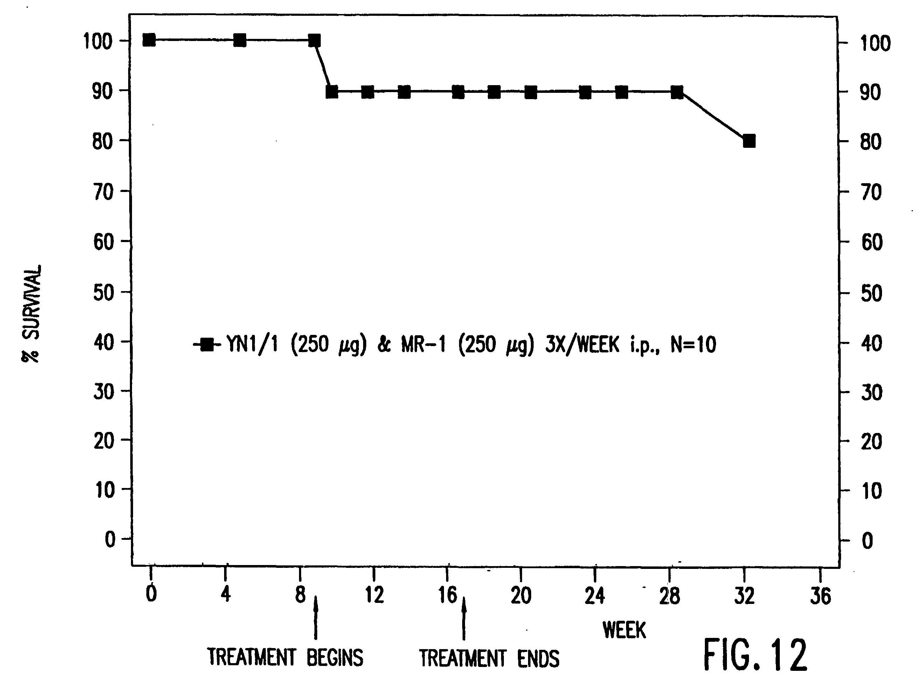

FIG. 12 depicts the effect of YN1/1 anti-ICAM-1 antibody and MR-1 anti-CD40L antibody on the % survival of (NZB/NZW)F₁ SLE mice in late stage disease. (■): YN1/1 anti-ICAM-1 antibody and MR-1 anti-CD40 ligand antibody (each antibody administered at 250 µg i.p. 3 times/week, N=10). Treatments were ended two months after they were begun.

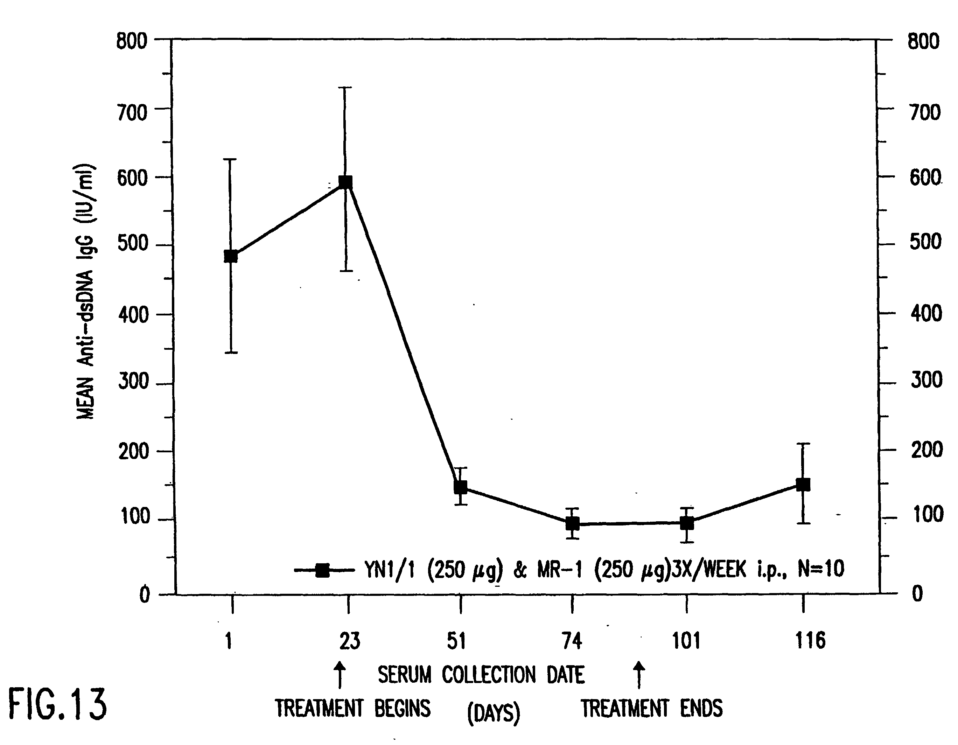

FIG. 13 depicts the effect of YN1/1 anti-ICAM-1 antibody and MR-1 anti-CD40L antibody on the mean level of anti-dsDNA IgG in (NZB/NZW)F₁ SLE mice in the late stage of the disease. (■): YN1/1 anti-ICAM-1 antibody and MR-1 1 anti-CD40 ligand antibody (each antibody administered at 250 µg i.p. 3 times/week, N=10). Treatments were ended two months after they were begun.

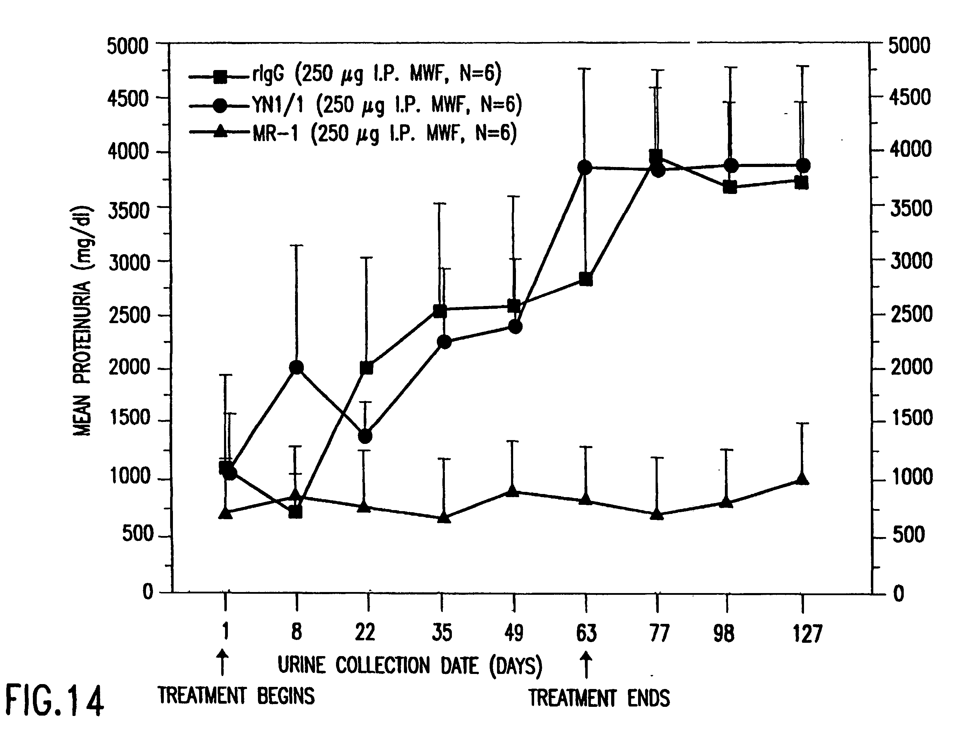

FIG. 14 depicts the effect of YN1/1 anti-ICAM-1 antibody and MR-1 anti-CD40L antibody on the mean level of proteinuria in (NZB/NZW)F₁ SLE mice in late stage disease. (■): rat IgG (250 µg i.p. MWF, N=6), (●): YN1/1 anti-ICAM-1 antibody (250 µg i.p. MWF, N=6); (▲): MR-1 anti-CD40 ligand antibody (250 µg i.p. MWF, N=6). Treatments were ended two months after they were begun.

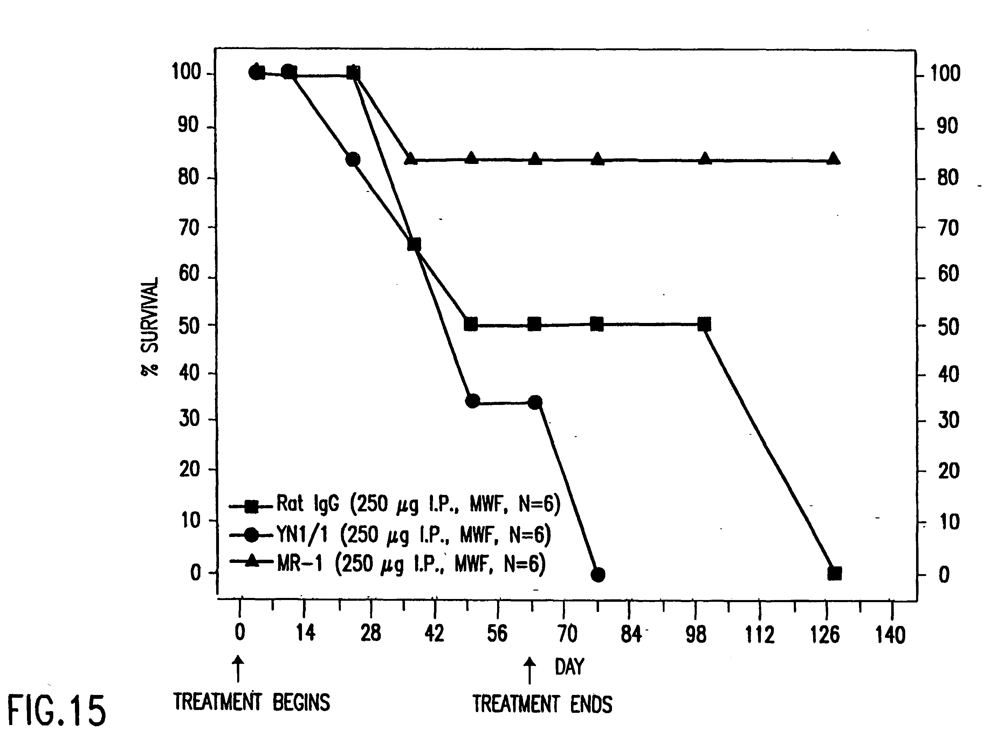

FIG. 15 depicts the effect of YN1/1 anti-ICAM-1 antibody and MR-1 anti-CD40L antibody on the % survival of (NZB/NZW)F₁ SLE mice in late stage disease. (■): rat IgG (250 µg i.p. MWF, N=6); (●): YN1/1 anti-ICAM-1 antibody (250 µg i.p. MWF, N=6); (▲): MR-1 anti-CD40 ligand antibody (250 µg i.p. MWF, N=6). Treatments were ended two months after they were begun.

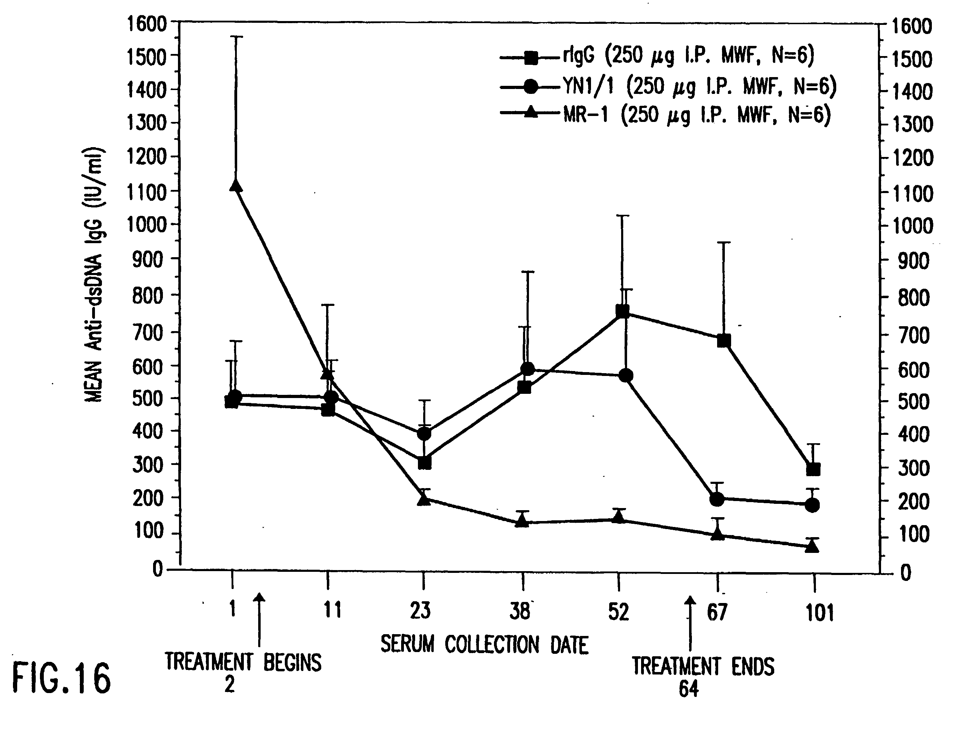

FIG. 16 depicts the effect of YN1/1 anti-ICAM-1 antibody and MR-1 anti-CD40L antibody on the mean level of anti-dsDNA IgG in (NZB/NZW)F₁ SLE mice in late stage disease. (■): rat IgG (250 µg i.p. MWF, N=6); (●): YN1/1 anti-ICAM-1 antibody (250 µg i.p. MWF, N=6); (▲): MR-1 anti-CD40 ligand antibody (250 µg i.p. MWF, N=6). Treatments were ended two months after they were begun.

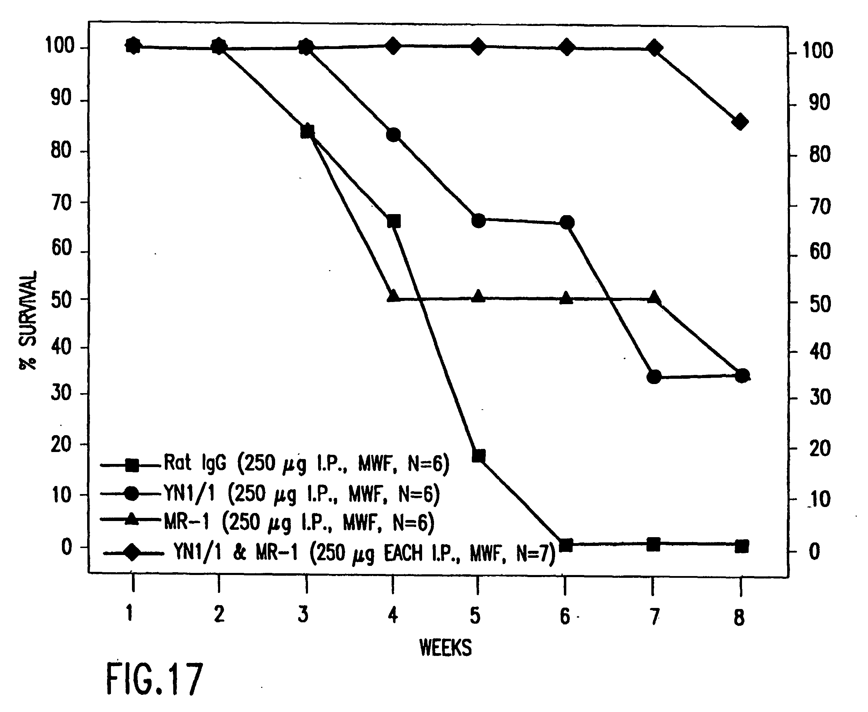

FIG. 17 depicts the effect of YN1/1 anti-ICAM-1 antibody and MR-1 anti-CD40L antibody on the % survival of (NZB/NZW)F₁ SLE mice in end stage disease (proteinuria was 2,000 - 4,000 mg/dl). (■): rat IgG (250 µg i.p. MWF, N=6); (●): YN1/1 anti-ICAM-1 antibody (250 µg i.p. MWF, N=6); (▲): MR-1 anti-CD40 ligand antibody (250 µg i.p. MWF, N=6); (◆): YN1/1 anti-ICAM-1 antibody and MR-1 anti-CD40 ligand antibody (each antibody administered at 250 µg i.p. MWF, N=7).

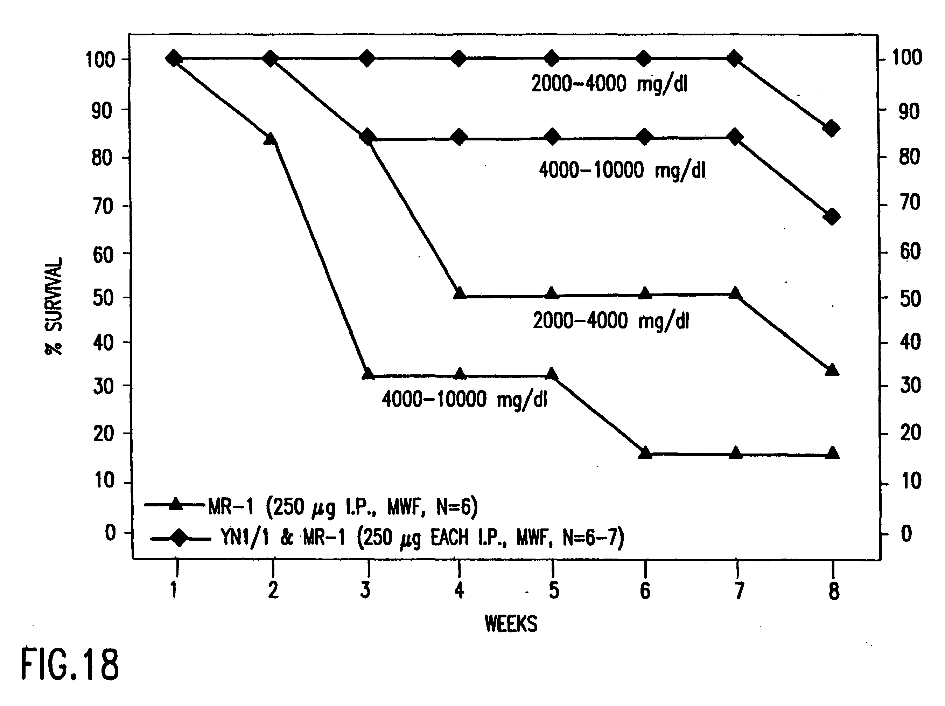

FIG. 18 depicts the effect of YN1/1 anti-ICAM-1 antibody and MR-1 anti-CD40L antibody on the % survival of(NZB/NZW)F₁ SLE mice in end stage disease. (▲): MR-1 anti-CD40 ligand antibody (250 µg i.p. MWF, N=6); (◆): YN1/1 anti-ICAM-1 antibody and MR-1 anti-CD40 ligand antibody (each antibody administered at 250 µg i.p. MWF, N=6-7).

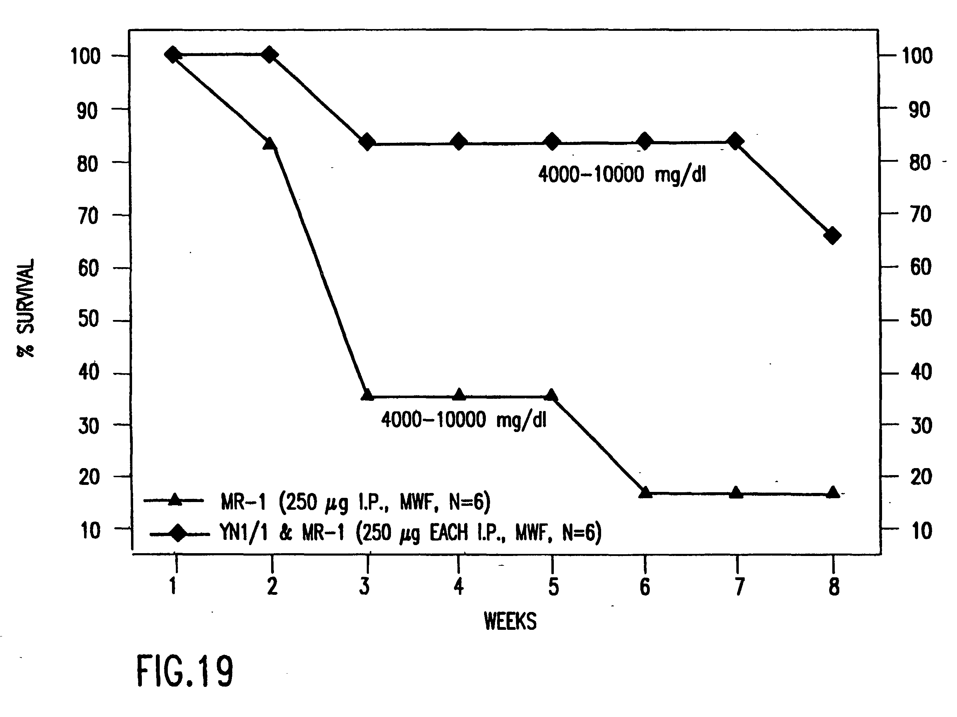

FIG. 19 depicts the effect of YN1/1 anti-ICAM-1 antibody and MR-1 anti-CD40L antibody combination therapy, versus MR-1 treatment alone, on the % survival of (NZB/NZW)F₁ SLE mice in end stage disease (proteinuria was 4,000 - 10,000 mg/dl). (▲): MR-1 anti-CD40 ligand antibody (250 µg i.p. MWF, N=6); (◆): YN1/1 anti-ICAM-1 antibody and MR-1 anti-CD40 ligand antibody (each antibody administered at 250 µg i.p. MWF, N=7).

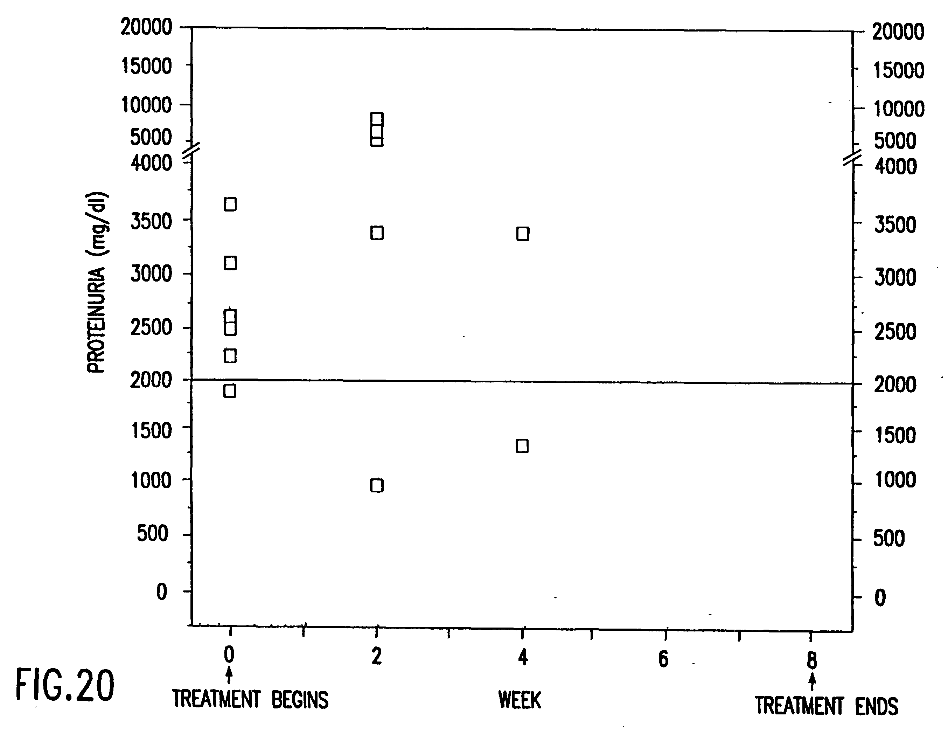

FIG. 20 depicts the effect of rat IgG mean level of proteinuria in (NZB/NZW)F₁ SLE mice in end stage disease. (□) Rat IgG antibody (250 µg i.p. MWF, N=6). Treatment began at time 0 and ended at week 8.

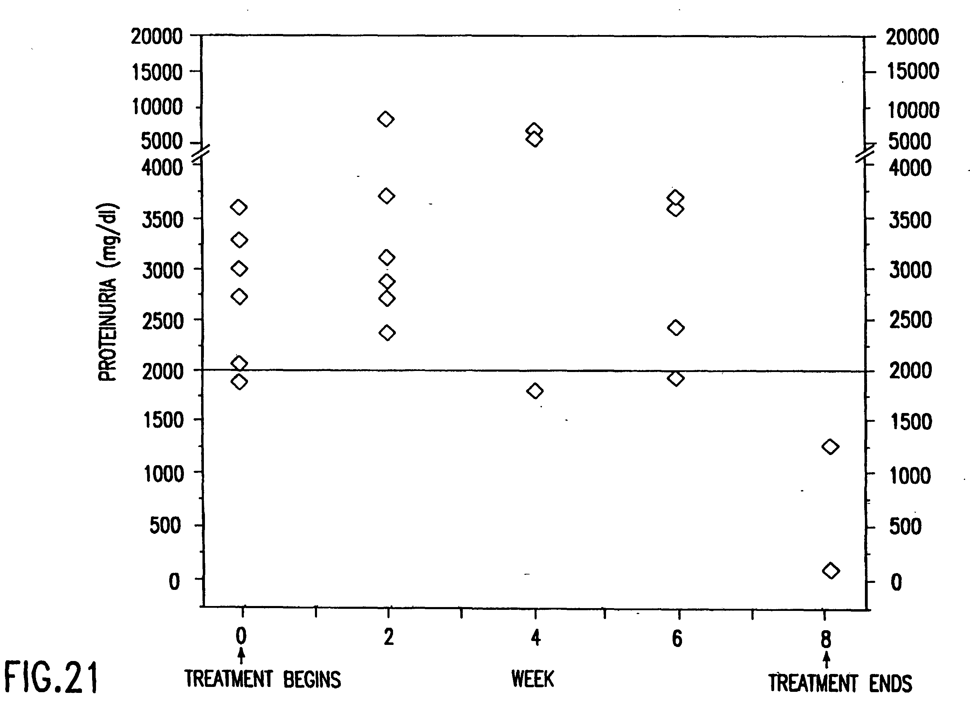

FIG. 21 depicts the effect of YN1/1 anti-ICAM-1 antibody on the mean level of proteinuria in (NZB/NZW)F₁ SLE mice in end stage disease. (◇) YN1/1 antibody (250 µg i.p. MWF, N=6). Treatment began at time 0 and ended at week 8.

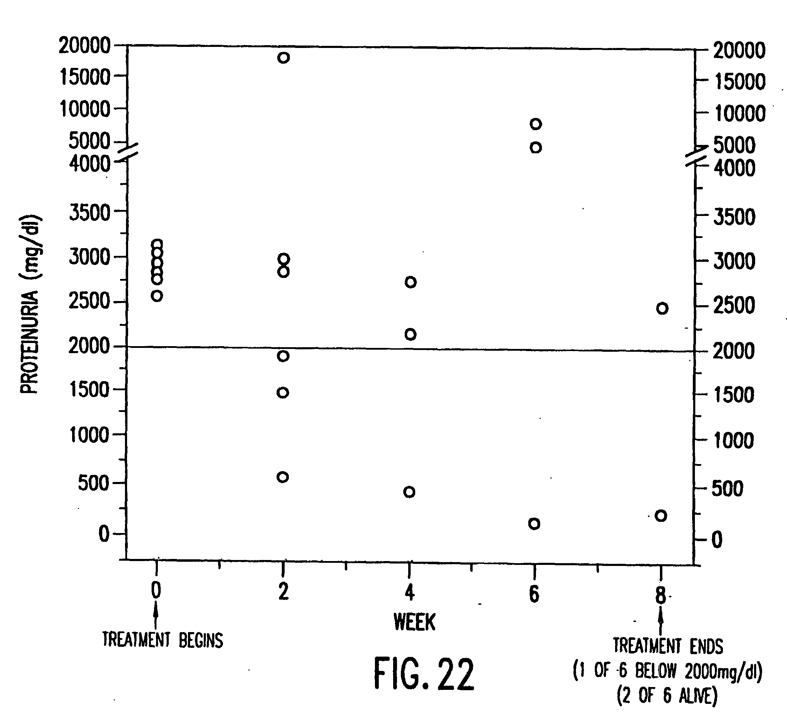

FIG. 22 depicts the effect of MR-1 anti-CD40L antibody on the mean level of proteinuria in (NZB/NZW)F₁ SLE mice in end stage disease. (○) MR-1 antibody (250 µg i.p. MWF, N=6). Treatment began at time 0 and ended at week 8. When treatment ended, two of the six animals were alive, and one of the six animals exhibited proteinuria at a level below 2,000 mg/dl.

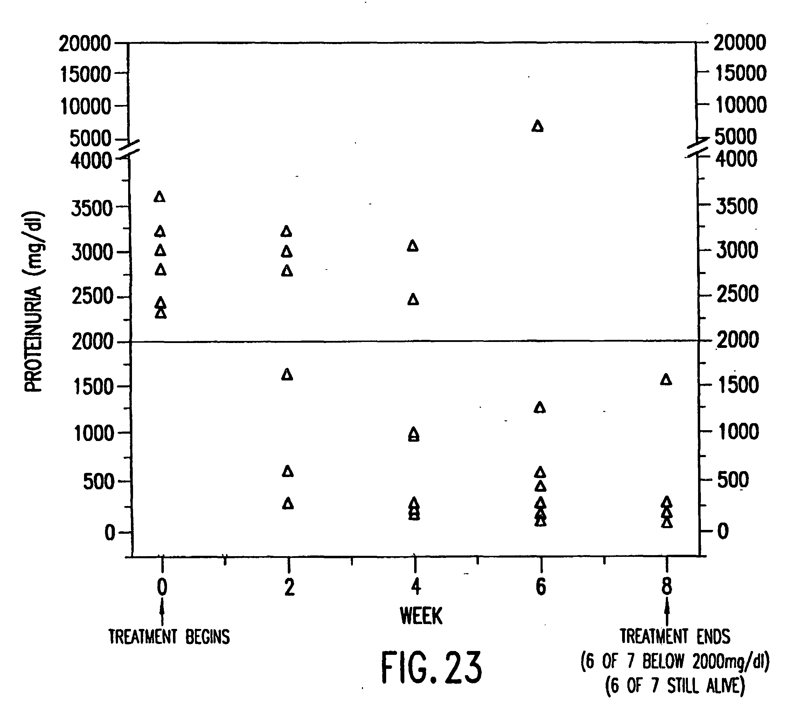

FIG. 23 depicts the synergistic effect of YN1/1 anti-ICAM-1 antibody and MR-1 anti-CD40L antibody in combination on the mean level of proteinuria in (NZB/NZW)F₁ SLE mice in end stage disease. (Δ) YN1/1 antibody and MR-1 antibody (each at 250 µg i.p. MWF, N=7). Treatment began at time 0 and ended at week 8. When treatment ended, six of the seven animals were alive, and exhibited proteinuria at a level below 2,000 mg/dl.

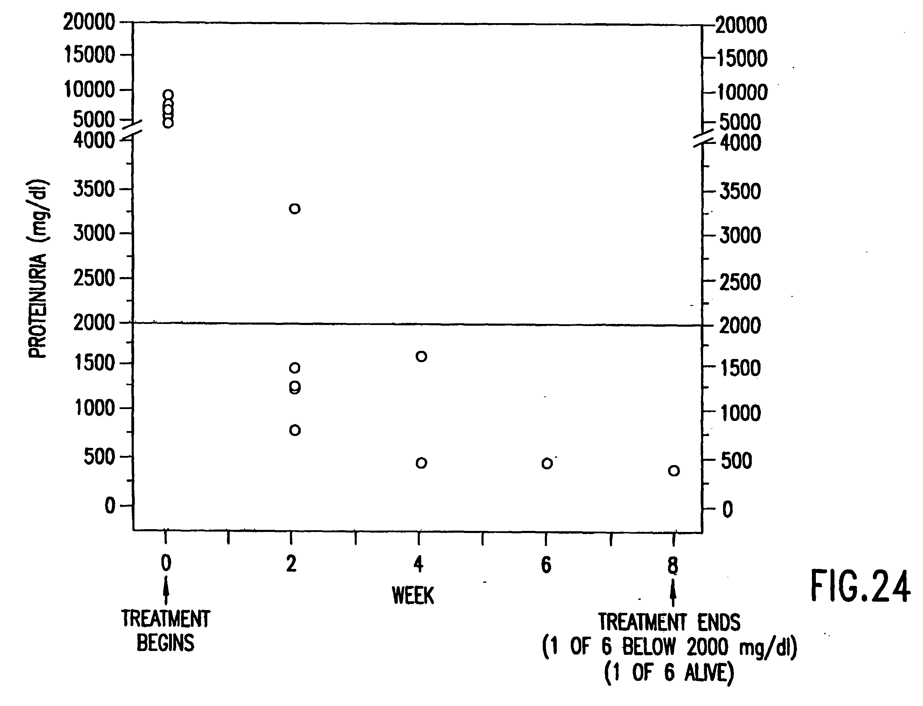

FIG. 24 depicts the effect of MR-1 anti-CD40L antibody on the mean level of proteinuria in (NZB/NZW)F₁ SLE mice in end stage disease, having a level of proteinuria of greater than 4,000 mg/dl per 24 hour urine sample (O) MR-1 antibody (250 µg i.p. MWF, N=6). Treatment began at time 0 and ended at week 8. When treatment ended, one of the six animals were alive, and exhibited proteinuria at a level below 2,000 mg/dl.

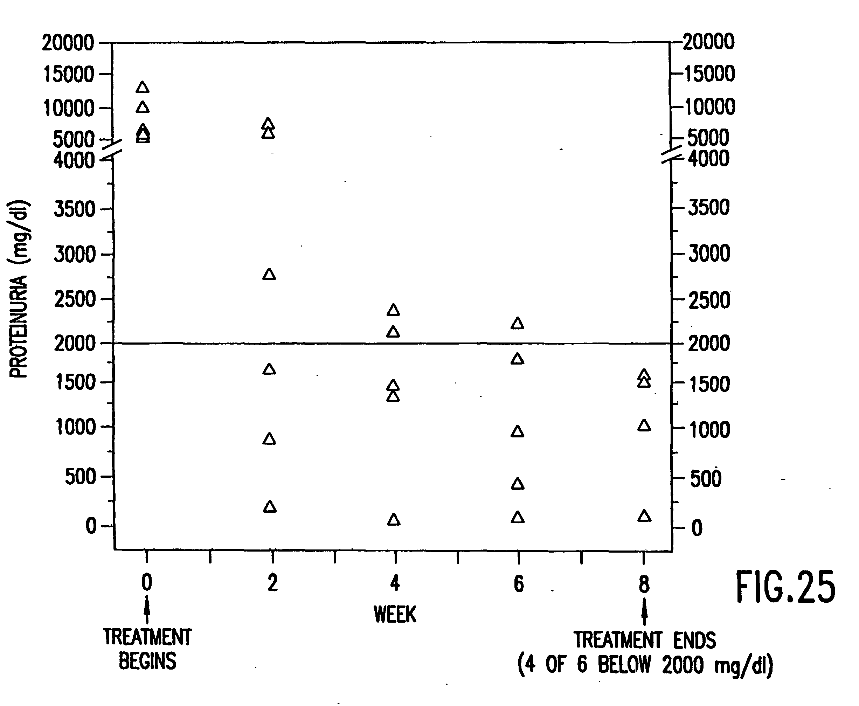

FIG. 25 depicts the effect of YN1/1 anti-ICAM- 1 antibody and MR-1 anti-CD40L antibody in combination on the mean level of proteinuria in (NZB/NZW)F₁ SLE mice in end stage disease, having a level of proteinuria of greater than 4,000 mg/dl per 24 hour urine sample. (Δ) YN1/1 antibody and MR-1 antibody (each at 250 µg i.p. MWF, N=6). Treatment began at time 0 and ended at week 8. When treatment ended, four of the seven animals were alive, and exhibited proteinuria at a level below 2,000 mg/dl.

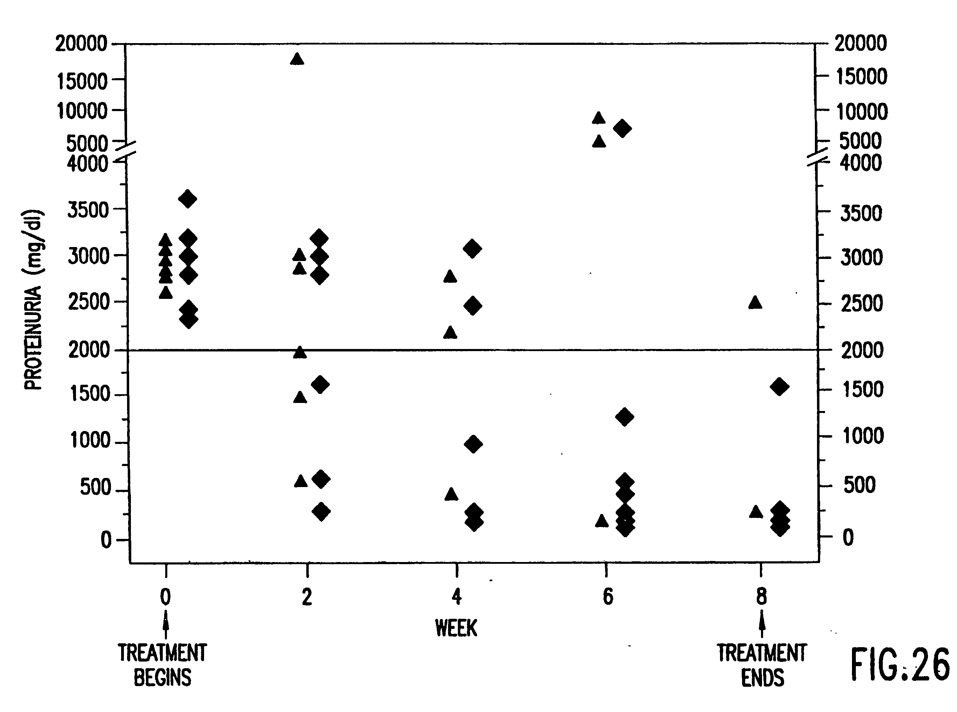

FIG. 26 depicts the comparison of the effect of anti-CD40L monotherapy with the effect of anti-ICAM-1/anti-CD40L combination therapy (NZB/NZW)F₁ SLE mice in end stage disease. Treatment began at time 0 and ended at week 8. The combination therapy resulted in prolonged survival and decreased proteinuria. (▲) MR-1 antibody (250 µg i.p. MWF, N=6). (◆) YN1/1 antibody and MR-1 antibody (each at 250 µg i.p. MWF, N=7).

### Detailed Description of the Invention

It has been found that coadministration of an agent that interrupts or perturbs ICAM-LFA-1 interaction and an agent that interrupts or perturbs CD40-CD40 ligand interaction provides a synergistic therapeutic effect. The present invention provides compositions and medicaments for treating autoimmune disease, particularly rheumatoid arthritis and systemic lupus erythematosus. Using the compositions and medicaments of the present invention, autoimmune diseases, and particularly rheumatoid arthritis and SLE, can be treated.

All scientific and technical terms used herein have meanings commonly used in the art, unless otherwise specified.

"Administering" refers providing one or more pharmaceutical agents to a subject. Thus, "administering" includes to oral adninisuation, administration as a suppository, topical contact, intravenously, intraperitoneally, intramuscnlarly, subcutaneously, intranasally, to the implantation of a slow-release device such as a miniosmotic pump, and to administration by inhalation.

In providing a mammal, and particularly a human, with therapeutic agents, the dosage will vary depending upon such factors as the recipient's age, weight, height, sex, general medical condition, previous medical history, etc. In general, it is desirable to provide the recipient with a dosage in the range of from about 1 pg/kg to 10 mg/kg (body weight of recipient), although a lower or higher dosage can be administered. Routes of administration can be intravenous, intramuscular, subcutaneously, intraperitoneal, enteral, or parenteral. The dosage frequency can be repeated at intervals ranging from each day to every other month.

Agents that regulate ICAM-LFA-1 interaction for use in the present invention are agents that interrupt, or perturb ICAM-LFA-1 interaction. Preferably, the ICAM is ICAM-1, ICAM-2, or ICAM-3. Still more preferably, the ICAM is ICAM-1.

Agents that regulate ICAM-LFA-1 interaction encompass any chemical and/or biological molecules that do not prevent ICAM and LFA-1 from binding to each other, but nonetheless prevent a subsequent physiological effect ofICAM-LFA-1 binding. Additionally, agents that interrupts or perturbs ICAM-LFA-1 interaction encompass any chemical and/or biological molecules that prevent ICAM and LFA-1 from binding to each other, block binding, or inhibit binding. More particularly, the agents block ICAM-LFA-1 interaction. Still more particularly, agents that interrupts or perturbs ICAM-LFA-1 interaction are polyclonal or monoclonal antibodies that bind to ICAM and/or LFA-1 and prevent ICAM and LFA-1 from binding to each other. Alternatively, agents that interrupts or perturbs ICAM-LFA-1 interaction are non-antibody compounds, such as small molecule antagonists, that bind to ICAM and/or LFA-1 and prevent ICAM and LFA-1 from binding to each other.

Soluble ICAM-1 derivatives are also encompassed by the phrase "agents that interrupts or perturbs ICAM-LFA-1 interaction." Soluble ICAM-1 derivatives are derivatives which are not bound to a membrane of a cell. Such derivatives may comprise truncated molecules which lack a transmembrane domain. Alternatively, they may comprise mutant forms of the natural molecules which lack the capacity to be bound (or stably bound) to the membrane of a cell even though they contain a transmembrane domain. Soluble derivatives of ICAM-1 and their preparation are disclosed by Marlin, S. D. *et al., Nature 344*:70-72 (1990). Among the preferred functional derivatives of ICAM-1 are soluble fragments of the ICAM-1 molecule which contain domains 1, 2, and 3 of ICAM-1. More preferred are soluble fragments of the ICAM-1 molecule which contain domains 1 and 2 of ICAM-1. Most preferred are soluble fragments of the ICAM-1 molecule which contain domain 1 of ICAM-1. See U. S. Patent No. 5,248,931.

Examples of LFA-1 small molecule antagonists include, but are not limited to, the molecules disclosed in international patent application no. PCT/US98/04254 (WO 98/39303) and international patent application no. PCT/EP98/05415 (WO 99/11258). In the methods and compositions of the present invention, preferred are molecules having structure (I) depicted in international patent application no. PCT/US98/04254 (WO 98/39303).

Especially preferred are the molecule depicted in example 271 (at page 221), having the structure 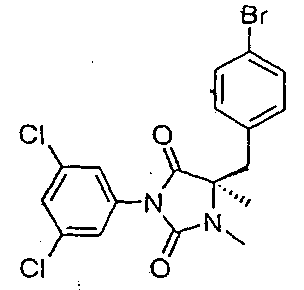 and the molecule of example 102 (at page 125) of international patent application no. PCT/US98/04254 (WO 98/39303), having the structure

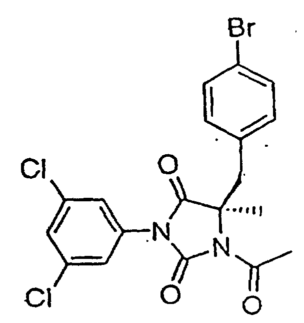

Those of ordinary skill in the art can determine whether an agent regulates ICAM-1-LFA-1 interaction without undue experimentation. For example, an *in vitro* assay of ICAM-1-LFA-1 interaction is provided in U.S. Patent No. 5,284,931.

Agents that interrupts or perturbs CD40-CD40 ligand interaction for use in the present invention are agents that interrupt, or perturb CD40-CD40 ligand interaction. Additionally, agents that interrupts or perturbs CD40-CD40 ligand interaction encompass any chemical and/or biological molecules that prevent CD40 and CD40 ligand from binding to each other, block binding, or inhibit binding. More particularly, the agents block CD40-CD40 ligand interaction. Still more particularly, agents that interrupts or perturbs CD40-CD40 ligand interaction are polyclonal or monoclonal antibodies that bind to CD40 and/or CD40 ligand and prevent CD40 and CD40 ligand from binding to each other. Alternatively, agents that interrupts or perturbs CD40-CD40 ligand interaction are non-antibody compounds, such as small molecule antagonists, that bind to CD40 and/or CD40 ligand and prevent CD40 and CD40 ligand from binding to each other.

Those of ordinary skill in the art can determine whether an agent interrupts or perturbs CD40-CD40 ligand interaction without undue experimentation using *in vitro* assays. Such assays are disclosed, for example, in U.S. Patent No. 5,683,693, 5,833, 987, 5,869,049, U.S. Patent Nos. 5,916,560, and international patent application no. PCT/US97/00668 (WO 97/26000).

"Anti-ICAM1 antibody" refers to an antibody that specifically recognizes and binds to an ICAM. Preferably, the antibody binds to ICAM-1, ICAM-2 or ICAM-3. Most preferably, the antibody binds to ICAM-1. Polyclonal and/or monoclonal antibodies can be used in the present invention. Anti-ICAM-1 antibodies can be made and used by those of ordinary skill in the art without undue experimentation. See U.S. Patent No. 5,284,931. Examples of anti-ICAM-1 antibodies include, but are not limited to, monoclonal antibody R6-5-D6 (ATCC 9580), and monoclonal antibody YN1/1 (ATCC CRL-1878). A fragment of a complete anti-ICAM-1 antibody, which retains the activity of the complete anti-ICAM-1 antibody, is also suitable in the methods and compositions of the present invention. An active fragment of a complete anti-ICAM-1 antibody retains the activity of the complete antibody if the fragment regulates ICAM-1-LFA-1 interaction.

"Anti-LFA-1 antibody" refers to an antibody that specifically recognizes and binds to LFA-1. Polyclonal and/or monoclonal antibodies can be used in the present invention. Anti-LFA-1 antibodies can be made and used by those of ordinary skill in the art without undue experimentation. For example, see U.S. Patent No. 5,284,931. Examples ofanti-LFA-1 antibodies include, but are not limited to monoclonal antibody M17/4.4 (ATCC TIB-217), monoclonal antibody TS2/18.1.1 (ATCC HB-195), monoclonal antibody TS 1/22.1.1.13 (ATCC HB-202), monoclonal antibody TS 1/18.1.2.11 (ATCCHB-203), monoclonal antibody LM2/1.6.11 (ATCC HB-204), monoclonal antibody TS2/9.1.4.3 (ATCC HB-205), monoclonal antibody 2E6 (ATCC HB-226), monoclonal antibody BE29G1 (ATCC HB-233), monoclonal antibody TS2/16.2.1 (ATCC HB-243), monoclonal antibody TS2/4.1.1 (ATCC HB-244), monoclonal antibody TS2/7.1.1 (ATCC HB-245), monoclonal antibody S6F1 (ATCC HB-9579), monoclonal antibody M5/114.15.2 (ATCC TIB-120), monoclonal antibody M1/70.15.11.5.HL (ATCC TIB-128), monoclonal antibody FD441.8 (ATCC TIB-213), monoclonal antibody M17/4.4.11.9 (ATCC TIB-217), monoclonal antibody M18/2.a.12.7 (ATCC TIB-218), monoclonal antibody M17/5.2 (ATCC TIB-237), and monoclonal antibody M5/49.4.1 (ATCC TIB-238).

A fragment of a complete anti-LFA-1 antibody, which retains the activity of the complete anti-LFA-1 antibody, is also suitable in the present invention. An active fragment of a complete anti-LFA-1 antibody retains the activity of the complete antibody if the fragment regulates ICAM-1-LFA-1 interaction.

"Anti-CD40 antibody" refers to an antibody that specifically recognizes and binds to CD40. Anti-CD40 antibodies can be made and used by those of ordinary skill in the art without undue experimentation. See U.S. Patent No. 5,801,227. A fragment of a complete anti-CD40 antibody, which retains the activity of the complete anti-CD40 antibody, is also suitable in the present invention. An active fragment of a complete anti-CD40 antibody retains the activity of the complete antibody if the fragment regulates CD40-CD40 interaction.

"Anti-CD40 ligand antibody" (anti-CD40L antibody) refers to an antibody that specifically recognizes and binds to CD40 ligand. Anti-CD40 ligand antibodies can be made and used by those of ordinary skill in the art without undue experimentation. See U.S. Patent No. 5,683,693, 5,833, 987, 5,869,049, U.S. Patent Nos. 5,916,560, and international patent application no. PCT/US97/00668 (WO 97/26000). Examples of anti-CD40 ligand antibodies include, but are not limited to, Examples of anti-CD40 ligand antibodies include, but are not limited to, Genzyme (Cambridge, MA) anti-CD40 ligand (product no. 80-3702-01), mouse anti-human CD40 ligand antibodies 24-31, 89-79, 89-76, 24-43, 409-8, and 409-9 monoclonal antibody 5c8 (ATCC no. HB 10916), monoclonal antibody MR-1 (ATCC no. HB-11048), and monoclonal antibody BG9588 (see National Institutes of Health Protocol Number: 99-AR-0133), monoclonal anti-human CD40 ligand MK13A4 (Pullen *et al., J. Biol. Chem.* (1999), from Alexis Biochemicals. In a preferred embodiment, the anti-CD40 ligand antibody is BG9588.

A fragment of a complete anti-CD40 ligand antibody, which retains the activity of the complete anti-CD40 ligand antibody, is also suitable for use in the present invention. An active fragment ofa complete anti-CD40 ligand antibody retains the activity of the complete antibody ifthe fragment regulates CD40-CD40 ligand interaction.

The medicaments and compositions of the present invention can be made and practiced using any kind of suitable antibodies, including polyclonal, monoclonal, humanized, chimeric, or primatized antibodies.

A chimeric antibody is an antibody in which the light and/or heavy chains contain regions from different species. For example, one or more variable (V) region segments of one species may be joined to one or more constant (C) region segments of another species. Typically, a chimeric antibody contains variable region segments of a mouse joined to human constant region segments, although other mammalian species may be used.

A humanized antibody is an antibody comprising one or more complementarity determining regions (CDRs) of a non-human antibody functionally joined to human framework regions segments. Additional residues associated with the non-human antibody can optionally be present. Typically, at least one heavy chain or one light chain comprises non-human CDRs. Typically, the non-human CDRs are mouse CDRs.

A primatized antibody is an antibody comprising one or more CDRs of an antibody of a species other than a non-human primate, functionally joined to framework region segments of a non-human primate. Additional residues associated with the species from which the CDR is derived can optionally be present. Typically, at least one heavy chain or one light chain comprises CDRs of the species which is not a nonhuman primate.

The production of polyclonal, monoclonal humanized, chimeric, or primatized antibody is well-known to those of ordinary skill in the art. For example, see *Antibodies: A Laboratory Manual,* E. Harlow, Ed., Cold Spring Harbor Laboratory Press (1998); Vaswani, S.K. *et al., Ann. Allergy, Asthma & Immunol. 81*: 105-115 (1998); Cuoto,.R. *et al., Cancer Res. (Suppl.) 55*: 5973s-5977s (1995); and U.S. Patent No. 5,714,350.

"Active fragment" refers to a portion of a complete antibody that provides the same physiological effect, and particularly the same therapeutic effect, as the complete antibody. The effect provided by the active fragment can be greater or less than the effect provided by the complete antibody.

"Autoimmune disease" diseases occur when immune reactivity to antigen arises through a breakdown in the mechanisms which control tolerance. Nonlimiting examples of autoimmune disease are rheumatoid arthritis, atopic dermatitis, any form of lupus (including cutaneous lupus and discoid lupus erythematosis), and any extracutaneous type of lupus (including systemic lupus erythematosus, acute lupus, lupus annularis, lupus discretus, lupus lymphaticus, lupus papollamtis, lupus psoriasis, lupus vulgaris, lupus sclerosis, neonatal lupus erythematosus and drug-induced lupus), alopecia areata, ankylosing spondylitis, antiphospholipid syndrome, autoimmune Addison's Disease, autoimmune hemolytic anemia, autoimmune hepatitis, Bechet's Disease, bullous pemphieiod, cardiomyopathy, celiac sprue-dermatitis, chronic fatigue immune dysfunction syndrome, chronic inflammatory demyelinating polyneuropathy, Churg-Strauss syndrome, circatricial pemphigiod, CREST syndrome, cold agglutinin disease, discoid lupus, essential mixed cryoglobulinemia, fibromyalgia fibromyositis, Grave's Disease, Guillain-Barré, hemolytic anemia, Hashimoto's thyroiditis, idiopathic pulmonary fibrosis, idiopathic thrombocytopenia, idiopathic thrombocytopenia purpura, IgA nephropathy, insulin-dependent diabetes, juvenille arthritis, lichen planus, lupus, Ménière's Disease, mixed connective tissue disease, multiple sclerosis, myasthenia gravis, pemphigus vulgaris, pernicious anemia, polyarteritis nodosa, polychondritis, polyglandular syndrome, polymyalgia rheumatica, polymyositis and dermatomyositis, primary agammaglobulinemia, primary biliary cirrhosis, psoriasis, Raynaud's phenomenon, Reiter's syndrome, rheumatic fever, sarcoidosis, scleroderma, Sjörgren's syndrome, stiff-man syndrome, thyroiditis, inflammatory bowel disease, Crohn's disease, Takayasu arteritis, temporal arteritis, giant cell arteritis, ulcerative colitis, uveitis, vasculitis, vitiligo, and Wegener's granulomatosis.

Preferably,"autoimmune disease" is selected from the group consisting of rheumatoid arthritis and systemic lupus erythematosus. Most preferably, "autoimmune disease" is either rheumatoid arthritis or systemic lupus erythematosus.

"CD40-CD40 ligand interaction" refers to binding between CD40 and CD40 ligand. One known ligand for CD40 is CD154 (gp39). See U.S. Patent No. 5,916,560. The terms "CD40 ligand," "CD154," "gp39," "CD40L,""T-BAM" and "TRAP" are synonymous.

"End-stage" systemic lupus erythematosus is characterized in part by nephritis, in which patients exhibit persistent urine proteinuria of greater than 1 gram/24 hour urine sample. More particularly, end-stage systemic lupus erythematosus is characterized by persistent urine proteinuria of greater than 1.5 grams/24 hour urine sample. More particularly, end-stage systemic lupus erythematosus is characterized by persistent urine proteinuria of greater than 2 grams/24 hour urine sample. More particularly, end-stage systemic lupus erythematosus is characterized by persistent urine proteinuria of greater than 2.5 grams/24 hour urine sample.

"ICAM-1-LFA-1 interaction" refers to binding between ICAM-1 and LFA-1. The terms "ICAM-1" and "intracellular adhesion molecule-1" are synonymous. The terms "LFA-1" and "leukocyte factor adhesion-1" are synonymous. ICAM-1-LFA-1 interaction can be detected and measured *in vitro* using techniques that are well-known to those of ordinary skill in the art. For example, see U. S. Patent No. 5,284,931.

The term "mammal" includes, dogs, cats, cows, horses, mice, rats, gerbils, guinea pigs, ferrets, and primates (humans, apes, chimpanzees, gorillas, and monkeys). In a preferred embodiment, the mammal is a human.

In the uses of the present invention, the synergistic ratio of (i) an agent that interrupts or perturbs ICAM-1-LFA-1 interaction, and (ii) an agent that interrupts or perturbs CD40-CD40 ligand interaction can be provided to the subject either individually, or together in the same (i.e., unitary) pharmaceutical composition. A unitary composition is a pharmaceutical composition that comprises (i) an agent that interrupts or perturbs ICAM-1-LFA-1 interaction, and (ii) an agent that interrupts or perturbs CD40-CD40 ligand interaction. Naturally, the unitary composition can be administered as often as deemed necessary to achieve the desired therapeutic effect.

"Pharmaceutically acceptable carrier" includes any material which when combined with a therapeutic agent, retains the therapeutic agent's activity and is nonreactive with the mammal's immune system. Examples include, but are not limited to, any of the standard pharmaceutical carriers such as a phosphate buttered saline solution, water, emulsions such as oil/water emulsion, and various types of wetting agents. Other carriers may also include sterile solutions, tablets, including coated tablets, and capsules. Typically, such carriers contain excipients, such as starch, milk, sugar certain types of clay, gelatin, stearic acid or salts thereof, magnesium or calcium stearate, talc, vegetable fats or oils, gums, glycols, or other known excipients. Such carriers may also include flavor and color additives or other ingredients. Compositions comprising such carriers are formulated by well known conventional methods.

The compositions of the present invention can be formulated according to known methods to prepare pharmaceutically useful compositions, whereby these materials, or their functional derivatives, are combined in admixture with a pharmaceutically acceptable carrier vehicle. Suitable vehicles and their formulation, are described, for example, in Remington's Pharmaceutical Sciences, 18^{th} edition, A. R Gennaro, Ed., Mack Publ., Easton, PA (1990).

Additional pharmaceutical methods may be employed to control the duration of action. Control release preparations may be achieved through the use of polymers to complex or absorb therapeutic agents. The controlled delivery may be exercised by selecting appropriate macromolecules (for example polyesters, polyamino acids, polyvinyl, pyrrolidone, ethylenevinylacetate, methylcellulose, carboxymethylcellulose, or protamine, sulfate) and the concentration of macromolecules as well as the methods of incorporation in order to control release. Another possible method to control the duration of action by controlled release preparations is to incorporate the therapeutic agent(s) into particles of a polymeric material such as polyesters, polyamino acids, hydrogels, poly(lactic acid) or ethylene vinylacetate copolymers. Alternatively, instead of incorporating these agents into polymeric particles, the therapeutic agent(s) can be entrapped in microcapsules prepared, for example, by coacervation techniques or by interfacial polymerization, for example, hydroxymethylcellulose or gelatine-microcapsules and poly(methylmethacylate) microcapsules, respectively, or in colloidal drug delivery systems, for example, liposomes, albumin microspheres, microemulsions, nanoparticles, and nanocapsules or in macroemulsions. Such techniques are disclosed in Remington's Pharmaceutical Sciences, 18^{th} edition, A. R. Gennaro, Ed., Mack Publ., Easton, PA (1990).

"Simultaneous administration" encompasses coadministration of at least two therapeutic agents, regardless of the relative frequencies or timing of the administration of the respective agents. Thus, simultaneous administration encompasses the coadministration of at least two therapeutic agents at the same time and at the same frequencies of administration. In addition, simultaneous administration refers to the coadministration of at least two therapeutic agents, in which one agent is administered more frequently than the other(s). In addition, simultaneous administration refers to the coadministration of at least two therapeutic agents, in which one agent is administered only once during the administration of the other agent(s).

In the present invention, other agents can be administered in addition to (i) the agent that interrupts or perturbs ICAM-1-LFA-1 interaction, and (ii) the agent that interrupts or perturbs CD40-CD40 ligand interaction

"Synergistic effect" refers, *in vitro*, a synergistic effect is one that is greater than the additive effect that would be predicted by summing the actual effects of the individual agents *in vitro. In vivo,* a synergistic effect is a physiological effect, and particularly a therapeutic effect, that is greater than the additive effect that would be predicted by summing the actual effects of the individual agents *in vivo*.

Thus, if two agents are administered, they together provide a measurable physiological effect, and particularly a therapeutic effect, if the actual effect of the agents together is greater than would be predicted by summing the actual therapeutic effects of the individual agents. More particularly, a synergistic effect is provided when a first agent alone provides no measurable effect, a second agent alone provides some measurable effect, and together the two agents provide a measurable effect greater than the effect provided by the second agent alone. Still more particularly, a synergistic effect is provided when neither a first agent alone nor a second agent alone provide any measurable effect, but together the two agents provide a measurable effect.

"Synergistic ratio" refers to a dosage ratio of therapeutic agents that provides a synergistic effect. A synergistic ratio of therapeutic agents can be determined by one of ordinary skill in the art without undue experimentation. See Chou, Synergism and Antagonism in Chemotherapy, Acad. Press, San Diego, pp. 61-102(1991). For example, efficacy, pharcnacokinetics, and pharmacodynamics can be determined for the various ratios. Various ratios of dosages can be administered to test subjects, until one or more of the tested ratios is found that provides a synergistic effect.

The medicaments and compositions of the present invention can be used to decrease the severity of an established disease. For example, in rheumatoid arthritis, the medicaments and compositions of the present invention can be used to decrease edema in one or more affected joints, decrease pain in one or more affected joints, or increase mobility in one or more affected joints. In systemic lupus eyrethematosus, the medicaments and compositions of the present invention can be used to of decrease proteinuria (measured in a 24 hour urine specimen), slow the progression of the patients' proteinuria, or halt the progression of the patients' proteinuria.

"Therapeutic effect" refers to a measurable and clinically beneficial effect provided to a patient to whom therapeutic agents are administered, relative to the effect obtained in subject to whom the agents are not provided. Thus, a subject to whom the agents are administered will experience improvement in his or her autoimmune disease, or at least prevention of further disease progression. For example, subject with rheumatoid arthritis will experience one or more of a decrease in pain, an increase in mobility and/or ability to bear weight on an affected joint, a decrease in joint edema, or at least no further worsening of the arthritis. A subject with end-stage systemic lupus erythematosus will experience a decrease in proteinuria (measured in a 24 hour urine specimen), or at least no further worsening of proteinuria, or a decrease in the level of anti-IgG double-stranded DNA antibodies.

In a preferred embodiment of the present invention, the agent that interrupts or perturbs the ICAM-1-LFA-1 interaction and the agent that interrupts or perturbs the CD40-CD40 ligand interaction are administered simultaneously.

While the medicaments and compositions of the present invention are suitable for treating autoimmune disease generally, they are particularly suitable for treating rheumatoid arthritis and systemic lupus erythematosis. The medicaments and compositions ofthe present invention are still more suitable for treating end-stage systemic lupus erythematosis.

The combination therapy provided by the medicaments and composition of the present invention can be augmented by the administration of one or more immunosuppressive agents, such as cyclosporin. For a list of immunosuppressive agents, see Gilman, A. G. *et al., Goodman and Gilman's the Pharmacological Basis of Therapeutics,* 9th Edition, McGraw-Hill Book Company (1995).

The compositions of the present invention can also be used to treat organ transplant patients in a wide variety of tissue and organ transplant situations. There are certain situations, such as with an allogeneic transplant or in "graft versus host" disease, where it would be extremely useful to suppress the immune response in order to prevent the rejection of helpful foreign tissue or organs. Allogeneic tissues and organs are tissues and organs from a genetically different member of the same species. "Graft versus host" disease occurs where the transplanted tissue, for example in a bone marrow transplant, contains allogeneic T-cells of the donor which cause an immune response against the recipient's own tissues. Although both humoral and cell-mediated immune responses play a role in the rejection of allogeneic tissues and organs, the primary mechanism involved is the cell-mediated immune response. Suppression of the immune response, and in particular, suppression ofcell-mediated immune response, would thus be useful in preventing such rejection of allograft tissues and organs. The compositions of the present invention can be used to induce T cell tolerance in a recipient of a graft of a tissue or organ such as pancreatic islets, liver, kidney, heart, lung, skin, muscle, neuronal tissue, stomach and intestines.

Having generally described the invention, the same will be more readily understood by reference to the following examples, which are provided by way of illustration and are not intended as limiting.

### Example 1

### Collagen-Induced Arthritis Model Of Rheumatoid Arthritis

Collagen-induced arthritis (CIA) has been described as an animal model for studying potential drugs or biologies active in human rheumatoid arthritis (Nabozny, G.H. *et al., Autoimmunity 20*: 39-49 (1995); *Guidance for Industry, Clinical Development Prograins for Drugs, Devices, and Biological Products for the Treatment of Rheumatoid Arthritis (RA)*, U.S. Dept. Health & Human Services (1999)). In the following examples, the role of the ICAM-1/LFA-1 and CD40/CD40L regulation pathways were studied in the model of established CIA.

Unless described otherwise, studies using the CIA animal model were performed as follows. B 1 0.RIII male or female mice were obtained from the Jackson Laboratory (Bar Harbor, ME) and were 10-12 weeks old at the beginning ofthe experiments. Animals were identified by subcutaneous microchips and were maintained in accordance with BIACUC guidelines. To induce arthritis, lyophilized native porcine type II collagen (CII) was dissolved overnight at 4°C in 0.01 N acetic acid at a concentration of 2 mg/ml. The collagen solution was then emulsified at a 1:1 ratio with complete Freund's adjuvant (containing 2 mg/ml *Mycobacterium tuberculosis* strain H37Ra). All animals received a single intradermal injection of 100 µl (containing 100 µg type II collagen) of cold emulsion in the base of the tail.

Beginning 12-14 days after immunization, all animals were monitored daily for signs of clinical arthritis, as indicated by limb erythema and edema. Arthritic animals were randomly enrolled into study groups until a total of 7-10 animals/group was obtained. Upon enrollment, an animal received treatment, as outlined below, for a period of 35 days (five weeks). Antibodies that were given 3 times per week were given on Monday (M), Wednesday (W) and Friday (F).

Following enrollment, all animals were monitored 3 times per week for arthritis severity. The extent of arthritis per paw was graded on a scale of 0-4 as follows: 0 = normal, 1 = erythema and edema in 1-3 digits; 2 = severe edema and erythema encompassing the tarsal and metatarsal joints; 3 = joint deformity along with edema and erythema encompassing the tarsal and metatarsal joints; 4 = joint ankylosis. The clinical score per paw was summed to give a maximal possible score of 16 per animal.

At enrollment and/or five weeks post-treatment, all animals were bled and the level of porcine type II collagen specific IgG in the sera was determined by ELISA. 100 µl of collagen coating solution (10 µg collagen/ml in 0.15 M KPO₄ (pH 7.6)) was added to each well of prechilled plates on ice. The coated plates were covered, incubated overnight at 4°C, and then washed with cold P/N/T buffer (phosphate-buffered saline/0.05% Tween 20/0.2 M NaCl). 300 µl of cold blocking buffer (P/N/T /1% bovine serum albumin) was added to each well while the plates were on ice. The plates were covered, incubated overnight at 4°C, and washed with cold P/N/T buffer. 100 µl of diluted sera and sera test samples was added to duplicate wells. Negative (nonimmunized) and positive (known) sera controls were placed in the appropriate wells. The plates were covered, incubated overnight at 4°C, and washed with cold P/N/T buffer.

At room temperature, 100 µl of conjugate solution (peroxidase-conjugated IgG fraction of goat anti-rat or mouse CII IgG (heavy and light chain specific) (Organon Technica, Durham, NC) was added to each well, the plates were covered, and incubated in the dark for one hour. After washing three times with P/N/T buffer, 100 µl of substrate solution (O-phenylenediamine/0.05 M citrate, pH 5.0) was added to each well, and the plates were incubated in the dark for 15-20 minutes. To stop the reactions, 50 µl of 2.5 N H₂SO₄ was added to each well. Absorbance was measured at 492 nm in a Titertek reader.

Edema caused by an inflammatory response was measured by plethysometry. Mean mercury (Hg) displacement in an assay of inflammation based on paw volume. The live animal paw dipped, up to the lateral malleolus, into a beaker filled with mercury, and the mean displacement volume plus or minus the standard error was measured.

YN1/1, M17/4.4, and MR-1 myeloma clones were obtained from American Type Tissue Collection, and antibodies were prepared.

Differences in arthritic severity between groups was determined using either the Wilcoxon-Mann Whitney test or the student's t-test. Differences in the level of type II collagen IgG and the extent of paw edema were determined using the student's t-test.

### Example 2

The effect of anti-CD40 ligand (CD40L) antibody and anti-ICAM-1 antibody combination therapy was compared to the effect of betamethasone therapy. In the studies from which the data in FIGS. 1A and I B were obtained, eight to ten male animals with established CIA were used for each respective treatment. In FIG. 1A, the mean arthritis score is shown (± standard error) over time for each respective treatment. (■): rat IgG (250 µg i.p. 3 times/week); (▼): YN1/1 anti-ICAM-1 antibody (250 µg i.p. 3 times/week); (▲): beta-methasone (5 mg/kg/i.p. every day); (●): phosphate-buffered saline (0.2 ml i.p. every day); (◆): MR-1 anti-CD40 ligand antibody (250 µg i.p. 3 times/week); ( 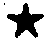 ): YN1/1 anti-ICAM-1 antibody (250 µg i.p. 3 times/week)/ MR-1 anti-CD40 ligand antibody (250 µg i.p. 3 times/week). * p < 0.05 vs. rat IgG and PBS controls.

Anti-CD40L antibody treatment alone did not alter CIA progression, relative to the control (PBS or rat IgG) treatments. In contrast, anti-ICAM-1 antibody treatment alone showed a significant inhibition of CIA severity. Anti-CD40L antibody and anti-ICAM-1 antibody combination therapy provided a synergistic effect, at all time points.

FIG 1B depicts the level of type II collagen IgG 5 weeks after the indicated treatments. Anti-CII IgG was assayed in an ELISA assay, and is shown as the mean units peroxidase activity (OD=492 nm) per ml X 10⁻³± standard error. Anti-CD40L antibody treatment significantly modulated antibody production, relative to the rat IgG control. * p < 0.05 vs. rat IgG control. In contrast, anti-ICAM 1 antibody treatment alone had no effect on type II collagen IgG antibody levels. Combination therapy significantly inhibited antibody type II collagen IgG antibody levels more effectively than either monotherapy. ** p < 0.05 vs. rat IgG and phosphate-buffered saline (PBS) controls.

### Example 3

For the combination therapy, the effect of anti-ICAM-1 antibody dosage was determined. In the studies from which the data in FIGS. **2A-G** were obtained, nine to ten male animals with established CIA were used for each respective treatment.

FIG. 2A depicts the effect of anti-CD40L antibody and of anti-ICAM-1 antibody dosage on the mean arthritic severity score. The mean arthritis severity score is shown (± standard error) over time for each respective treatment. (■): rat IgG (250 µg i.p. 3 times/week); (●): MR-1 anti-CD40 ligand antibody (250 µg i.p. 3 times/week); (▲): YN1/1 anti-ICAM-1 antibody (250 µg i.p. 3 times/week); (▼): YN1/1 anti-ICAM-1 antibody (125 µg i.p. 3 times/week); (◆): YN1/1 anti-ICAM-1 antibody (50 µg i.p. 3 times/week).

MR-1 treatment alone yielded an effect that was significantly different from rat IgG control only at week 3. YN-1/1 treatment alone (250 ug) was significantly different from the rIgG control at all time points YN-1/1 treatment alone (125 ug) was significantly different from the rIgG control at all time points but week 3. YN-1/1 treatment alone (50 ug) was significantly different from the rIgG control at weeks 2, 3, 4, but not weeks 1 and 5. In this dosage range of YN1/1, a dose-response was not obtained.

FIG. 2B depicts the synergistic effect of anti-CD40L antibody and of anti-ICAM-1 antibody dosage on the mean arthritic severity score for anti-CD40L and anti-ICAM-1 combination therapy. The mean arthritis severity score is shown (± standard error) over time for each respective treatment. (■): rat IgG (500 µg i.p. 3 times/week); (●): MR-1 anti-CD40 ligand antibody (250 µg i.p. 3 times/week); (▲): MR-1 anti-CD40 ligand antibody (250 µg i.p. 3 times/week) and YN1/1 anti-ICAM-1 antibody (250 µg i.p. 3 times/week); (▼): MR-1 anti-CD40 ligand antibody (250 µg i.p. 3 times/week) and YN1/1 anti-ICAM-1 antibody (125 µg i.p. 3 times/week); (◆): MR-1 anti-CD40 ligand antibody (250 µg i.p. 3 times/week) and YN1/1 anti-ICAM-1 antibody (50 µg i.p. 3 times/week). FIG. 2B shows that all doses of anti-ICAM-1 antibody were equally effective.

FIG. 2C depicts the synergistic effect of anti-CD40L antibody and of anti-ICAM-1 antibody dosage on the mean arthritic severity score for anti-CD40L and anti-ICAM-1 combination therapy. In FIG. 2C, whereas the (●) data represent the effect of combination therapy for five doses, (▲), (▼), and (◆) data represent the effect of combination therapy (different anti-ICAM-1 antibody dosages) for 5 weeks. The data obtained after five dosages revealed a delay in disease progression, and not a long-lasting immune response.

The mean arthritis severity score is shown (± standard error) over time for each respective treatment. (■): rat IgG (500 µg i.p. Q.I.D., MWF for 5 weeks); (●): MR-1 anti-CD40 ligand antibody (250 µg i.p. Q.I.D., MWF for 1 week); (▲): MR-1 anti-CD40 ligand antibody (250 µg i.p. Q.I.D., MWF for 5 weeks) and YN1/1 anti-ICAM-1 antibody (250 µg i.p. Q.I.D., MWF for 5 weeks); (▼): MR-1 anti-CD40 ligand antibody (250 µg i.p. Q.I.D., MWF for 5 weeks) and YN1/1 anti-ICAM-1 antibody (125 µg i.p. Q.I.D., MWF for 5 weeks); (◆): MR-1 anti-CD40 ligand antibody (250 µg i.p. Q.I.D., MWF for 5 weeks) and YN1/1 anti-ICAM-1 antibody (50 µg i.p. Q.I.D., MWF for 5 weeks).

### Example 4

FIG. 2D depicts the effect of anti-ICAM-1 antibody or anti-CD40L antibody on mean mercury (Hg) displacement in an assay of inflammation based on paw volume. Edema caused by an inflammatory response was measured by plethysometry. The live animal paw dipped, up to the lateral malleolus, into a beaker filled with mercury, and the mean displacement volume plus or minus the standard error was measured. (■): rat IgG (250 µg i.p. Q.I.D., MWF); (●): MR-1 anti-CD40 ligand antibody (250 µg i.p. Q.I.D., MWF); (A): YN1/1 anti-ICAM1 antibody (250 µg i.p., Q.I.D., MWF); (▼): YN1/1 anti-ICAM1 antibody (125 µg i.p., Q.I.D., MWF); (◆): YN1/1 anti-ICAM1 antibody (50 µg i.p., Q.I.D., MWF). The MR-1 antibody was not significant at any time point. While all YN1/1 dosages yielded a significant effect at week 1, only the 250 ug dose of YN1/1 yielded a significant effect at week 3, and none of the YN1/1 dosages yielded a significant effect at week 5.

FIG. 2E depicts the synergistic effect of anti-ICAM-1 antibody and anti-CD40L antibody on mean mercury (Hg) displacement in an assay of inflammation based on paw volume. (■): rat IgG (500 µg i.p.); (●): MR-1 anti-CD40 ligand antibody (250 µg i.p.) and YN1/1 anti-ICAMI antibody (250 µg i.p.) for five doses; (▲): MR-1 anti-CD40 ligand antibody (250 µg i.p.) and YN1/1 anti-ICAM1 antibody (250 µg i.p.) for five weeks; (▼): MR-1 anti-CD40 ligand antibody (250 µg i.p.) and YN1/1 anti-ICAM1 antibody (125 µg i.p.) for five weeks; (◆): MR-1 anti-CD40 ligand antibody (250 µg i.p.) and YN1/1 anti-ICAM1 antibody (50 µg i.p.) for five weeks. All MR-1/YN1/1 combinations yielded a synergistic effect, compared to the effect of the MR-1 antibody alone or YN1/1 antibody alone (see FIG. 2D).

FIG. 2F depicts the synergistic inhibition of established arthritis through anti-ICAM-1 and anti-CD40L combination therapy. The mean arthritic score is shown at enrollment and week 5 (+ standard error) foreach respective treatment. * p < 0.05 vs. rat IgG controls.

FIG. 2G depicts the synergistic inhibition of established edema through anti-ICAM-1 and anti-CD40L combination therapy. The mean Hg displacement is shown at enrollment and week 5 (+ standard error) for each respective treatment. * p < 0.05 vs. rat IgG controls.

Unlike prophylactic treatment, which effectively inhibits arthritis induction, therapeutic treatment (treatment of established disease) with anti-CD40L did not alter CIA progression. Anti-CD40L treatment did, however, affect the humoral response by decreasing antibody levels to type II collagen at week five, compared to rat IgG controls. In contrast, mice treated with anti-ICAM-1 alone showed a significant inhibition of CIA severity, but without affecting type II collagen IgG levels.

Remarkably, animals that received combination treatment with anti-ICAM-1 and anti-CD40L showed no progression of CIA over the 5-week period and, in some cases, showed reversal of the arthritis observed at the time of enrollment. Surprisingly, combination treatment (anti-ICAM-1 and anti-CD40L) inhibited the type II collagen-specific antibody levels more effectively than either treatment alone.

### Example 5

In the studies from which the data in FIGS. 3A-B were obtained, ten male animals with established CIA were used for each respective treatment.

FIG. 3A depicts the effect of anti-CD40L antibody and LFA-1 antagonist combination therapy on the mean arthritic severity score. LFA-1 "A" is an LFA-1 antagonist and is the compound synthesized in Example 271 at page 221 of international patent appl. no. PCT/US98/04254 (WO 98/39303). The structure of LFA-1 antagonist "A" is:

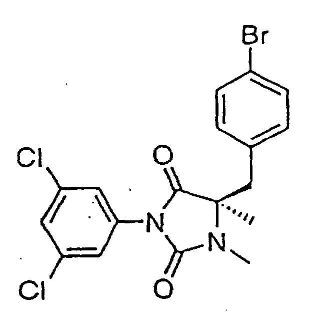

The mean arthritis severity score is shown (± standard error) over time for each respective treatment. (▲): olive oil control (100 µl P.O. b.i.d.); (●): LFA-1 antagonist "A" (30 mg/kg P.O. b.i.d.); (■): LFA-1 antagonist "A" (30 mg/kg P.O. b.i.d.) and MR-1 anti-CD40L antibody (250 µg i.p. 3X/week). LFA-1 antagonist "A" alone was not effective. However, in combination, LFA-1 antagonist "A" and MR-1 anti-CD40 ligand antibody treatment was significantly different than the olive oil control.

FIG 3B. is a bar-graph depicting the level of type II collagen IgG 5 weeks after the indicated treatments. Anti-CII IgG was assayed in an ELISA assay, and is shown as the mean units peroxidase activity (OD=492 nm) per ml X 10⁻³ ± standard error. After five weeks of treatment with olive oil (control), animals exhibited an 11.1% decrease in CII IgG. After five weeks of treatment with LFA-1 antagonist "A", animals exhibited a 1.6% increase in CII IgG. After five weeks of combination therapy with treatment with LFA-1 antagonist "A" and MR-1 anti-CD40L antibody, animals exhibited a 65.2% decrease in CII IgG. LFA-1 antagonist "A" alone was not effective. However, in combination, LFA-1 antagonist "A" and MR-1 anti-CD40 ligand antibody treatment was significantly different than the olive oil control.

### Example 6

FIG. 4 depicts the synergistic inhibition of established arthritis through anti-LFA-1 and anti-CD40L combination therapy. The mean arthritic score is shown (+ standard error) for each respective treatment. (▲): mAb M17/4.4 rat anti-mouse LFA-1 (250 µg i.p. 3X/week); (●): mAb MR-1 hamster anti-CD40 ligand antibody (250 µg i.p. 3X/week); (▼): combination M17/4.4 +MR-1 (250 µg + 250 µg i.p. 3X/week). Control mice received: (■): rat IgG (250 µg i.p. 3X/week); * p < 0.05 vs. rat IgG controls. Neither MR-1 anti-CD40 ligand antibody treatment alone nor M17/4.4 anti-LFA-1 antibody treatment alone was effective. However, combination therapy provided a synergistic effect.

### Comparative Example 7

This is a comparation example.

FIG. 5 depicts the synergistic effect of anti-ICAM-1 antibody and cyclosporin on the mean arthritic severity score. Seven to nine female animals with established CIA were used for each respective treatment. The mean arthritis severity score is shown (± standard error) over time for each respective treatment. (■): rat IgG (50 µg i.p., Q.I.D., MWF); (●): olive oil (100 µl P.O., B.I.D.); ( 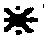 ): cyclosporin 30 mg/kg P.O., B.I.D.); (▼): YN1/1 anti-ICAM-1 antibody (50 µg i.p., Q.I.D., MWF); (▲):cyclosporin 30 mg/kg P.O., B.I.D.) and YN1/1 anti-ICAM-1 antibody (50 µg i.p., Q.I.D., MWF). Cyclosporin provided a significant effect at week 5. The effect of YN1/1 (50 pg) alone was significantly different form the effect of rat IgG at weeks 3, 4 and 5. A synergistic effect was observed at all time points for the combinaiton therapy.

FIG. 6 depicts the effect of LFA-1 antagonist and cyclosporin combination therapy on the mean arthritic severity score. LFA-1 "B" is an LFA-1 antagonist and is the compound synthesized in Example 102 at page 125 of international patent appl. no. PCT/US98/04254 (WO 98/39303). The structure of LFA-1 antagonist "B" is:

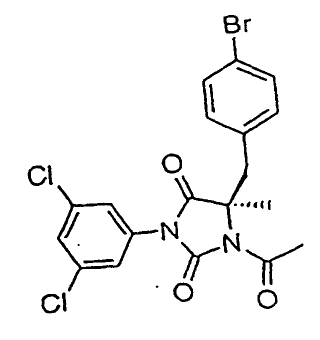

The mean arthritis severity score is shown (± standard error) over time for each respective treatment. (▲): olive oil control (100 µl P.O. b.i.d.); (■): LFA-1 antagonist "B" (50 mg/kg P.O. b.i.d.); (●): cyclosporin (30 mg/kg P.O. b.i.d.); (▼): LFA-1 antagonist "B" (50mg/kgP.O. b.i.d.) and cyclosporin (30 mg/kg P.O. b.i.d.). A synergistic effect was observed at weeks 3, 4 and 5.

### Example 8

### (NZB/NZW)F₁ Mouse Model of Systemic Lupus Erythematosus

The (NZB/NZW)F₁ mouse strain spontaneously develops an autoimmune disease with a course that is similar to systemic lupus erythematosus (SLE), and the strain has been described as an animal model of SLE (Ye, Y.-L. and B.-L. Chiang, *Clin. Exp. Rheum. 16*: 33-37 (1998)). The (NZB/NZW)F₁ strain spontaneously develops IgG anti-dsDNA antibodies, a characteristic oflupus, and develop proteinuria, which leads to increasing degrees of nephritis. (NZB/NZW)F₁ mice usually die early of uremia or complete kidney failure (*Clin. Exp. Rheum. 16:* 33-37 (1998)).

The production of autoantibodies, deposition of immune complexes and activation of the complement system are all early events leading to nephritis in lupus patients. The infiltration of inflammatory cells and their interaction with resident renal cells is involved in the progression of renal injury, and the amplification of inflammatory responses in lupus nephritis (See Belmont, H.M., "Lupus Nephritis: Treatment Issues," http://cerebel.com/lupus/nephritis.html (August 28, 1999)).

Proteinuria is generally accepted as a marker of disease progression *(Arthritis Advisory Committee, Design and Assessment of Clinical Trials of Drugs, Biologics and Devices That Are Being Developed for Treatment of Systemic Lupus Erythematosus*, U.S. Dept. Health & Human Services (1999)).

In the following examples, the role of the ICAM-1 and CD40L regulation pathways was studied during early-, late-, and end-stage disease in (NZB/NZW)F₁ lupus-prone mice.

Disease in the (NZB/NZW)F₁ strain can be categorized as "early-," "late-," or "end-stage," depending on the amount degree of proteinuria observed in the animal. Early stage refers to (NZB/NZW)F₁ mice at age 4-5 months. At,that age, mice generally exhibit a quantitative (24 hour) proteinuria of less than 300 mg/dl protein in a 24 hour urine sample, and a detectable level of anti-double-stranded DNA antibodies (approximate range from 50-300 IU/ml).

Late stage refers to (NZB/NZW)F₁ mice at age 6-7 months. At that age, mice generally exhibit a quantitative (24 hour) proteinuria of greater than 300 mg/dl protein in a 24 hour urine sample, and increased levels of anti-double-stranded DNA antibodies (approximate range from 300-1500 IU/ml).

End-stage refers to (NZB/NZW)F₁ mice with proteinuria of greater than 2,000 mg/dl urine in a 24 hour sample of urine.

### Example 9

### Materials and Methods

In the following examples, 3-6 week old female (NZB/NZW)F₁ mice were obtained from the Jackson Laboratory (Bar Harbor, ME). The animals were identified by subcutaneous microchips and were maintained in accordance with BIACUC guidelines until experimental use.

For studies in early-stage disease, animals were studied at age 4-5 months. 51 animals were randomly enrolled in groups of ten to eleven per treatment regimen. Groups 1-4 received treatments for a period of two months, and animals in group 5 did not receive treatment until age 6-7 months, the age at which the animals entered late stage disease. Animals were treated as indicated in Table 1.

**Table 1 -**

| **Early Stage Disease** | |
|---|---|
| Group | Treatment |
| 1 | rat IgG (250 µg i.p. 3 times/week) |
| 2 | YN1/1 anti-ICAM1 antibody (250 µg i.p. three times/week) |
| 3 | MR-1 antiCD40L antibody (250 µg i.p. three times/week) |
| 4 | YN1/1 (250 µg i.p. three times/week) and MR-1 (250 µg i.p. three times/week) |
| 5 | Late stage: YN1/1 (250 µg i.p. three times/week) and MR-1 (250 µg i.p. three times/week) |

For studies in late stage disease, 20 female 6 to 7 month old animals were randomly enrolled in groups of six animals per treatment regimen. Groups 1-3 received treatments for a period of two months, as indicated in Table 2.

**Table 2 -**

| **Late Stage Disease** | |
|---|---|
| Group | Treatment |
| 1 | rat IgG (250 µg i.p. 3 times/week) |
| 2 | YN1/1 anti-ICAM1 antibody (250 µg i.p. three times/week) |
| 3 | MR-1 antiCD40L antibody (250 µg i.p. three times/week) |

For studies in end-stage disease, 100 female 6-8 month old animals were used. A 24 hour urine sample was taken weekly, and total protein in the urine was quantified using an automated chemistry analyzer. After exhibiting a urine protein level of greater than or equal to 2,000 mg/dl for a 24 hour sample, animals were randomly enrolled in groups of six to seven for groups 1, 2, 3a and 4a. After exhibiting a urine protein level of greater than or equal to 4,000 mg/dl for a 24 hour sample, animals were randomly enrolled in groups of six to seven for groups 3b and 4b. Treatments were as shown in table 3.

**Table 3 -**

| **End Stage Disease** | |
|---|---|
| Group | Treatment |
| 1 | rat IgG (250 µg i.p. 3 times/week) |
| 2 | YN1/1 anti-ICAMI antibody (250 µg i.p. three times/week) |
| 3a | MR-1 antiCD40L antibody (250 µg i.p. three times/week) |
| 3b | MR-1 antiCD40L antibody (250 µg i.p. three times/week) |
| 4a | YN1/1 (250 µg i.p. three times/week) and MR-1 (250 µg i.p. three times/week) |
| 4b | YN1/1 (250 µg i.p. three times/week) and MR-1 (250 µg i.p. three times/week) |

In all studies, animals were housed in metabolism cages. Sera and a 24 hour urine sample were taken at enrollment and approximately every two weeks to monitor disease progression. Fresh urine samples were used to determine total protein in the urine was quantified using an automated Hitachi 911 chemistry analyzer.

Autoantibodies to double-stranded- (ds-) DNA in sera were determined using an ELISA assay. Blood samples were collected via tail vein into serum tubes, allowed to clot, and the serum was separated. Serum samples were stored undiluted at - 20°C until analyzed. Autoantibodies to dₛ-DNA in sera were determined by ELISA (The Binding Site, Birmingham, England, catalogue #MK017). Microwells were pre-coated with calf thymus dsDNA antigen. The calibrators, and diluted controls and mouse samples were added to the wells and autoantibodies recognizing the dsDNA antigen bind during the first incubation. After washing the wells to remove all unbound protein, purified peroxidase labeled rabbit ant-human IgG conjugate was added to the wells containing the calibrators and diluted controls. Purified peroxidase labeled goat anti-mouse IgG conjugate was added to the mouse sample wells. The conjugate bound to the captured human or mouse autoantibody, and the excess unbound conjugate was removed by a further wash step. The bound conjugate was visualized with tetramethylbenzidine (TMB) substrate, which gave a blue reaction product, the intensity of which was proportional to the concentration of autoantibody in the sample. Phosphoric acid was added to each well to stop the reaction. This produced a yellow end point color, which was quantified spectrophotometrically in a 96 well reader using 450 nm absorbance (Vmax, Molecular Devices).

### Example 10

FIG. 7 depicts the effect of various treatments on the mean proteinuria level in (NZB/NZW)F₁ SLE early stage mice. (■): rat IgG (250 µg i.p. MWF, N=8); (●): YN1/1 anti-ICAM-1 antibody (250 µg i.p. MWF, N=10); (▲): MR-1 anti-CD40 ligand antibody (250 µg i.p. MWF, N=10); (◆): YN1/1 anti-ICAM-1 antibody and MR-1 anti-CD40 ligand antibody (each antibody administered at 250 µg i.p. MWF, N=11); (▼): late stage: YN1/1 anti-ICAM-1 antibody and MR-1 anti-CD40 ligand antibody (each antibody administered at 250 µg i.p. MWF, N=10).

FIG. 8 depicts the effect of various treatments on the mean proteinuria level in (NZB/NZW)F₁ SLE mice in early stage disease. (■): rat IgG (250 µg i.p. MWF,N=8);(●): YN1/1 anti-ICAM-1 antibody (250 µg i.p. MWF, N=10); (▲): MR-1 anti-CD40 ligand antibody (250 µg i.p. NMRF, N=10); (◆): YN1/1 anti-ICAM-1 antibody and MR-1 anti-CD40 ligand antibody (each antibody administered at 250 µg i.p. MWF, N=11). Treatments were ended two months after they were begun.

FIG. 9 depicts the effect of various treatments on the % survival of (NZB/NZW)F₁ SLE mice in early stage disease. (■): rat IgG (250 µg i.p. MWF, N=10); (●): YN1/1 anti-ICAM-1 antibody (250 µg i.p. MWF, N=10); (▲): MR-1 anti-CD40 ligand antibody (250 µg i.p. MWF, N=10); (◆): YN1/1 anti-ICAM-1 antibody and MR-1 anti-CD40 ligand antibody (each antibody administered at 250 µg i.p. MWF, N=11). Treatments were ended two months after they were begun.

FIG. 10 depicts the effect of various antibody treatments on the mean level of anti-dsDNA IgG in (NZB/NZW)F₁ SLE mice in early stage disease. (■): rat IgG (250 µg i.p. MWF, N=10); (●): YN1/1 anti-ICAM-1 antibody (250 µg i.p. MWF, N=10); (▼): MR-1 anti-CD40 ligand antibody (250 µg i.p. MWF, N=10); (▲): YN1/1 anti-ICAM-1 antibody and MR-1 anti-CD40 ligand antibody (each antibody administered at 250 µg i.p. MWF, N=11). Treatments were ended two months after they were begun.

As shown in FIGS. 7-10, YN1/1 significantly delayed proteinuria and increased survival, versus rat IgG controls. However, YN1/1 had no effect on the level of double-stranded DNA antibodies. In contrast, MR-1 alone blocked proteinuria and MR-1 alone increased survival, compared to the YN1/1 alone treatment group and the rat IgG control group. MR-1 also blocked production of double-stranded DNA antibodies. This inhibitory effect seen with MR-1 alone was sustained well after treatment ended, unlike the effects seen in the group treated with YN1/1 alone.

### Example 11

FIG. 11 depicts the effect of YN1/1 anti-ICAM-1 antibody and MR-1 anti-CD40L antibody on the mean proteinuria level in (NZB/NZW)F₁ SLE mice in late stage disease. (■): YN1/1 anti-ICAM-1 antibody and MR-1 anti-CD40 ligand antibody (each antibody administered at 250 µg i.p. 3 times/week, N=10). Treatments were ended two months after they were begun. After examination of the proteinuria data, there was evidence that the combination therapy was able to effectively treat two mice with proteinuria > 2,000 mg/dl.

FIG. 12 depicts the effect of YN1/1 anti-ICAM-1 antibody and MR-1 anti-CD40L antibody on the % survival of (NZB/NZW)F₁ SLE mice in late stage disease. (■): YN1/1 anti-ICAM-1 antibody and MR-1 anti-CD40 ligand antibody (each antibody administered at 250 µg i.p. 3 times/week, N=10). Treatments were ended two months after they were begun.

FIG. 13 depicts the effect of YN1/1 anti-ICAM-1 antibody and MR-1 anti-CD40L antibody on the mean level of anti-dsDNA IgG in (NZB/NZW)F₁ SLE mice in late stage disease. (■): YN1/1 anti-ICAM-1 antibody and MR-1 anti-CD40 ligand antibody (each antibody administered at 250 µg i.p. 3 times/week, N=10). Treatments were ended two months after they were begun. FIGS. 12 and 13 show that in late disease, similar results as with MR-1 alone were obtained .

FIG. 14 depicts the effect of YN1/1 anti-ICAM-1 antibody and MR-1 anti-CD40L antibody on the mean level of proteinuria in (NZB/NZW)F₁ SLE mice in late stage disease. (■): rat IgG (250 µg i.p. MWF, N=6); (●): YN1/1 anti-ICAM-1 antibody (250 µg i.p. MWF, N=6); (▲): MR-1 anti-CD40 ligand antibody (250 µg i. p. MWF, N=6). Treatments were ended two months after they were begun. There was a single mouse in the MR-1 group with proteinuria > 2,000 mg/dl which was not treatable.

FIG. 15 depicts the effect of YN1/1 anti-ICAM-1 antibody and MR-1 anti-CD40L antibody on the % survival of (NZB/NZW)F₁ SLE mice in late stage disease. (■): rat IgG (250 µg i.p. MWF, N=6); (●): YN1/1 anti-ICAM-1 antibody (250 µg i.p. MWF, N=6); (▲): MR-1 anti-CD40 ligand antibody (250 µg i.p. MWF, N=6). Treatments were ended two months after they were begun.

FIG. 16 depicts the effect of YN1/1 anti-ICAM-1 antibody and MR-1 anti-CD40L antibody on the mean level of anti-dsDNA IgG in (NZB/NZW)F₁ SLE mice in late stage disease. (■): rat IgG (250 µg i.p. MWF, N=6);(●): YN1/1 anti-ICAM-1 antibody (250 µg i.p. MWF, N=6); (▲): MR-1 anti-CD40 ligand antibody (250 µg i.p. MWF, N=6). Treatments were ended two months after they were begun.

As shown in FIGS. 14-16, in late disease, YN1/1 had no effect on proteinuria, survival or antibody production. MR-1 blocked increased proteinuria, decreased antibodies to baseline, along with greatly improving survival. Again, these inhibitory effects were sustained throughout the study. In summary, while the ICAM-1 pathway played a significant role in the early disease progression, the CD40L pathway played a major role in both the early and late stages of disease.

FIG. 17 depicts the effect of YN1/1 anti-ICAM-1 antibody and MR-1 anti-CD40L antibody on the % survival of(NZB/NZW)F₁ SLE mice in end stage disease (proteinuria was 2,000 - 4,000 mg/dl). (■): rat IgG (250 µg i.p. MWF, N=6); (●): YN1/1 anti-ICAM-1 antibody (250 µg i.p. MWF, N=6); (▲): MR-1 anti-CD40 ligand antibody (250 µg i.p. MWF, N=6). (◆): YN1/1 anti-ICAM-1 antibody and MR-1 anti-CD40 ligand antibody (each antibody administered at 250 µg i.p. MWF, N=7). A synergistic effect was observed for the YN1/1 - MR-1 combination therapy.

FIG. 18 depicts the effect of YN1/1 anti-ICAM-1 antibody and MR-1 anti-CD40L antibody on the % survival of (NZB/NZW)F₁ SLE mice in end stage disease. (▲): MR-1 anti-CD40 ligand antibody (250 µg i.p. MWF, N=6); (◆): YN1/1 anti-ICAM-1 antibody and MR-1 anti-CD40 ligand antibody (each antibody administered at 250 µg i.p. MWF, N=6-7).

FIG. 19 depicts the effect of YN1/1 anti-ICAM-1 antibody and MR-1 anti-CD40L antibody combination therapy, versus MR-1 treatment alone, on the % survival of (NZB/NZW)F₁ SLE mice in end stage disease (proteinuria was 4,000 - 10,000 mg/dl). (▲): MR-1 anti-CD40 ligand antibody (250 µg i.p. MWF, N=6); (◆): YN1/1 anti-ICAM-1 antibody and MR-1 anti-CD40 ligand antibody (each antibody administered at 250 µg i.p. MWF, N=6).

FIG. 20 depicts the effect of rat IgG mean level of proteinuria in (NZB/NZW)F₁ SLE mice in end stage disease. (□) Rat IgG antibody (250 µg i.p. MWF, N=6). Treatment began at time 0 and ended at week 8. No animals were alive at 8 weeks.

FIG. 21 depicts the effect of YN1/1 anti-ICAM-1 antibody on the mean level of proteinuria in (NZB/NZW)F₁ SLE mice in end stage disease. (0) YN1/1 antibody (250 µg i. p. MWF, N=6). Treatment began at time 0 and ended at week 8. At week 8, one of the six animals were alive, and the proteinuria level was below 2,000 mg/dl.

FIG. 22 depicts the effect of MR-1 anti-CD40L antibody on the mean level of proteinuria in (NZB/NZW)F₁ SLE mice in end stage disease. (O) MR-1 antibody (250 µg i.p. MWF, N=6). Treatment began at time 0 and ended at week 8. When treatment ended, two of the six animals were alive, and one of the six animals exhibited proteinuria at a level below 2,000 mg/dl.

FIG. 23 depicts the synergistic effect of YN1/1 anti-ICAM-1 antibody and MR-1 anti-CD40L antibody in combination on the mean level of proteinuria in (NZB/NZW)F₁ SLE mice in end stage disease. (Δ) YN1/1 antibody and MR-1 antibody (each at 250 µg i.p. MWF, N=7). Treatment began at time 0 and ended at week 8. When treatment ended, six of the seven animals were alive, and exhibited proteinuria at a level below 2,000 mg/dl. Thus, the combination therapy resulted in prolonged survival and decreased proteinuria, versus YN1/1 antibody alone or MR-1 antibody alone.

FIG. 24 depicts the effect of MR-1 anti-CD40L antibody on the mean level of proteinuria in (NZB/NZW)F₁ SLE mice in end stage disease, having a level of proteinuria of greater than 4,000 mg/dl per 24 hour urine sample. (O) MR-1 antibody (250 µg i.p. MWF, N=6). Treatment began at time 0 and ended at week 8. When treatment ended, one of the six animals were alive, and exhibited proteinuria at a level below 2,000 mg/dl.

FIG. 25 depicts the effect of YN1/1 anti-ICAM-1 antibody and MR-1 anti-CD40L antibody in combination on the mean level of proteinuria in (NZB/NZW)F₁ SLE mice in end stage disease, having a level of proteinuria of greater than 4,000 mg/dl per 24 hour urine sample. (Δ) YN1/1 antibody and MR-1 antibody (each at 250 µg i.p. MWF, N=6). Treatment began at time 0 and ended at week 8. When treatment ended, four of the seven animals were alive, and exhibited proteinuria at a level below 2,000 mg/dl. Thus, the combination therapy resulted in prolonged survival and decreased proteinuria.

FIG. 26 depicts the comparison of the effect of anti-CD40L monotherapy with the effect of anti-ICAM-1/anti-CD40L combination therapy. Treatment began at time 0 and ended at week 8. The combination therapy resulted in prolonged survival and decreased proteinuria. (▲) MR-1 antibody (250 µg i.p. MWF, N=6). (◆) YN1/1 antibody and MR-1 antibody (each at 250 µg i.p. MWF, N=7).

In end-stage disease, where renal involvement and nephritis are established, anti-CD40L therapy was less effective. While a slight effect on survival was seen, compared to rat IgG controls (33% vs. 0%) at week 8, only one of the two surviving animals had decreased proteinuria (< 2000mg/dl) at week 8. Similar results were seen with anti-ICAM-1 therapy.

In contrast to monotherapy, synergistic effects were seen with anti-ICAM-1 and anti-CD40L combination therapy. Remarkable results were seen on survival, compared to rat IgG controls and monotherapies (85% vs. 0% and 33%, respectively) at week 8. All surviving animals exhibited decreased proteinuria (< 2000 mg/dl) at week 8.

Beneficial effects were also seen on survival in group 4b, where proteinuria was > 4000 mg/dl at enrollment, compared to anti-CD40L therapy (66% vs. 16%, respectively). All surviving animals exhibited decreased proteinuria (< 2000 mg/dl) at week 8.

Therefore, anti-ICAM-1 and anti-CD40L combination therapy, given after nephritis had been established, resulted in synergistic effects on survival and proteinuria, compared to either monotherapy.

It will be clear that the invention may be practiced otherwise than as particularly described in the foregoing description and examples.

Numerous modifications and variations of the present invention are possible in light of the above teachings and, therefore, are within the scope of the appended claims.

## Claims

1. A pharmaceutical composition for treating auto-immune disease, said composition comprising a pharmaceutically acceptable carrier and a synergistic ratio of (i) an agent that interrupts or perturbs ICAM-LFA-1 interaction, and (ii) an agent that interrupts or perturbs CD40-CD40 ligand interaction.

2. The composition of claim 1, wherein said ICAM is ICAM-1.

3. The composition as claimed in claim 1 or claim 2, wherein said auto-immune disease is rheumatoid arthritis or systemic lupus erythematosus.

4. The composition of claim 3, wherein said systemic lupus erythematosus is end-stage disease.

5. The composition as claimed in any one of claims 1 to 4, wherein said agent that interrupts or perturbs said ICAM-LFA-1 interaction is an antibody or an active fragment thereof.

6. The composition of claim 5, wherein said antibody is an anti-ICAM antibody or an active fragment thereof.

7. The composition of claim 6, wherein said anti-ICAM antibody is an anti-ICAM-1 antibody.

8. The composition of claim 1, wherein said agent that interrupts or perturbs said CD40-CD40 ligand interaction is an anti-CD40 antibody.

9. The composition of claim 1, wherein said agent that interrupts or perturbs said CD40-CD40 ligand interaction is an anti-CD40 ligand antibody or an active fragment thereof.

10. The composition as claimed in any one of claims 1-9, wherein said agent that interrupts or perturbs said ICAM-LFA-1 interaction and/or said agent that interrupts or perturbs said CD40-CD40 ligand interaction is a non-antibody agent.

11. The use of a pharmaceutical composition as claimed in any one of claims 1-10 in the manufacture of a medicament.

12. The use of a synergistic ratio of (i) an antibody that interrupts or perturbs ICAM-LFA-1 interaction, or an active fragment thereof, and (ii) an antibody that interrupts or perturbs CD40-CD40 ligand interaction, or an active fragment thereof in the manufacture of a medicament for use in the treatment of rheumatoid arthritis or systemic lupus erythematosus.

13. The use of claim 12, wherein said ICAM is ICAM-1.

14. The use of claim 12, wherein said antibody that interrupts or perturbs said ICAM-LFA-1 interaction is an anti-ICAM antibody, and wherein said antibody that interrupts or perturbs said CD40-CD40 ligand interaction is an anti-CD40 ligand antibody.

15. The use of claim 12, wherein said anti-ICAM antibody is an anti-ICAM-1 antibody.

16. The use of claim 12, wherein said antibody that interrupts or perturbs said ICAM-LFA-1 interaction is an anti-LFA-1 antibody or an active fragment thereof, and wherein said antibody that interrupts or perturbs said CD40-CD40 ligand interaction is an anti-CD40 ligand antibody or an active fragment thereof.

17. The use as claimed in any one of claims 12 to 16 wherein the systemic lupus erythematosus is end-stage.

18. The use as claimed in any one of claims 12-17, wherein said medicament further comprises agents other than (i) said agent that interrupts or perturbs ICAM-LFA-1 interaction, and (ii) said agent that interrupts or perturbs CD40-CD40 ligand interaction.

19. The use of a synergistic ratio of (i) an agent that interrupts or perturbs ICAM-LFA-1 interaction and (ii) an agent that interrupts or perturbs CD40-CD40 ligand interaction in the manufacture of a medicament for use in the treatment of an autoimmune disease.

20. The use as claimed in claim 19 wherein the autoimmune disease is rheumatoid arthritis or systemic lupus erythematosus.

21. A product comprising a synergistic ratio of (i) an agent that interrupts or perturbs ICAM-LFA-1 interaction and (ii) an agent that interrupts or perturbs CD40-CD40 ligand interaction as a combined preparation for simultaneous, sequential or separate use in treating autoimmune diseases.

## Patentansprüche

1. Pharmazeutische Zusammensetzung zur Behandlung einer Autoimmunerkrankung, wobei die Zusammensetzung einen pharmazeutisch akzeptablen Träger und ein synergistisches Verhältnis von (i) einem Mittel, das ICAM-LFA-1-Wechselwirkung unterbricht oder stört, und (ii) einem Mittel umfasst, das CD40-CD40-Ligand-Wechselwirkung unterbricht oder stört.

2. Zusammensetzung gemäß Anspruch 1, wobei das ICAM ICAM-1 ist.

3. Zusammensetzung gemäß Anspruch 1 oder 2, wobei die Autoimmunerkrankung rheumatoide Arthritis oder systemischer Lupus erythematodes ist.

4. Zusammensetzung gemäß Anspruch 3, wobei der systemische Lupus erythematodes eine terminale Erkrankung bzw. Erkrankung im Endstadium ist.

5. Zusammensetzung gemäß irgendeinem der Ansprüche 1 bis 4, wobei das Mittel, das die ICAM-LFA-1-Wechselwirkung unterbricht oder stört, ein Antikörper oder ein aktives Fragment davon ist.

6. Zusammensetzung gemäß Anspruch 5, wobei der Antikörper ein Anti-ICAM-Antikörper oder ein aktives Fragment davon ist.

7. Zusammensetzung gemäß Anspruch 6, wobei der Anti-ICAM-Antikörper ein Anti-ICAM-1-Antikörper ist.

8. Zusammensetzung gemäß Anspruch 1, wobei das Mittel, das die CD40-CD40-Ligand-Wechselwirkung unterbricht oder stört, ein Anti-CD40-Antikörper ist.

9. Zusammensetzung gemäß Anspruch 1, wobei das Mittel, das die CD40-CD40-Ligand-Wechselwirkung unterbricht oder stört, ein Anti-CD40-Ligand-Antikörper oder ein aktives Fragment davon ist.

10. Zusammensetzung gemäß irgendeinem der Ansprüche 1-9, wobei das Mittel, das die ICAM-LFA-1-Wechselwirkung unterbricht oder stört, und/oder das Mittel, das die CD40-CD40-Ligand-Wechselwirkung unterbricht oder stört, ein Nicht-Antikörper-Mittel ist.

11. Verwendung einer pharmazeutischen Zusammensetzung gemäß irgendeinem der Ansprüche 1-10 zur Herstellung eines Medikaments.

12. Verwendung eines synergistischen Verhältnisses von (i) einem Antikörper, der ICAM-LFA-1-Wechselwirkung unterbricht oder stört, oder einem aktiven Fragment davon, und (ii) einem Antikörper, der CD40-CD40-Ligand-Wechselwirkung unterbricht oder stört, oder einem aktiven Fragment davon zur Herstellung eines Medikaments zur Verwendung bei der Behandlung von rheumatoider Arthritis oder systemischen Lupus erythematodes.

13. Verwendung gemäß Anspruch 12, wobei das ICAM ICAM-1 ist.

14. Verwendung gemäß Anspruch 12, wobei der Antikörper, der die ICAM-LFA-1-Wechselwirkung unterbricht oder stört, ein Anti-ICAM-Antikörper ist, und wobei der Antikörper, der die CD40-CD40-Ligand-Wechselwirkung unterbricht oder stört, ein Anti-CD40-Ligand-Antikörper ist.

15. Verwendung gemäß Anspruch 12, wobei der Anti-ICAM-Antikörper ein Anti-ICAM-1-Antikörper ist.

16. Verwendung gemäß Anspruch 12, wobei der Antikörper, der die ICAM-LFA-1-Wechselwirkung unterbricht oder stört, ein Anti-LFA-1-Antikörper oder ein aktives Fragment davon ist, und wobei der Antikörper, der die CD40-CD40-Ligand-Wechselwirkung unterbricht oder stört, ein Anti-CD40-Ligand-Antikörper oder ein aktives Fragment davon ist.

17. Verwendung gemäß irgendeinem der Ansprüche 12 bis 16, wobei der systemische Lupus erythematodes terminal bzw. im Endstadium ist.

18. Verwendung gemäß irgendeinem der Ansprüche 12-17, wobei das Medikament weiterhin andere Mittel als (i) das Mittel, das ICAM-LFA-1-Wechselwirkung unterbricht oder stört, und (ii) das Mittel umfasst, das CD40-CD40-Ligand-Wechselwirkung unterbricht oder stört.

19. Verwendung eines synergistischen Verhältnisses von (i) einem Mittel, das ICAM-LFA-1-Wechselwirkung unterbricht oder stört und (ii) einem Mittel, das CD40-CD40-Ligand-Wechselwirkung unterbricht oder stört, zur Herstellung eines Medikaments zur Verwendung bei der Behandlung einer Autoimmunerkrankung.

20. Verwendung gemäß Anspruch 19, wobei die Autoimmunerkrankung rheumatoide Arthritis oder systemischer Lupus erythematodes ist.

21. Produkt, das ein synergistisches Verhältnis von (i) einem Mittel, das ICAM-LFA-1-Wechselwirkung unterbricht oder stört, und (ii) einem Mittel, das CD40-CD40-Ligand-Wechselwirkung unterbricht oder stört, als ein kombiniertes Präparat zur simultanen, sequentiellen oder getrennten Verwendung bei Behandlung von Autoimmunerkrankungen umfasst.

## Revendications

1. Composition pharmaceutique pour le traitement de maladie auto-immune, ladite composition comprenant un excipient pharmaceutiquement acceptable et un rapport synergique de (i) un agent qui interrompt ou perturbe l'interaction ICAM-LFA-1 et (ii) un agent qui interrompt ou perturbe l'interaction entre CD40 et son ligand.

2. Composition selon la revendication 1, dans laquelle ladite ICAM est ICAM-1.

3. Composition selon la revendication 1 ou la revendication 2, dans laquelle ladite maladie auto-immune est la polyarthrite rhumatoïde ou le lupus érythémateux disséminé.

4. Composition selon la revendication 3, dans laquelle ledit lupus érythémateux disséminé est une maladie en phase terminale.

5. Composition selon l'une quelconque des revendications 1 à 4, dans laquelle ledit agent qui interrompt ou perturbe ladite interaction ICAM-LFA-1 est un anticorps ou un fragment actif de celui-ci.

6. Composition selon la revendication 5, dans laquelle ledit anticorps est un anticorps anti-ICAM ou un fragment actif de celui-ci.

7. Composition selon la revendication 6, dans laquelle ledit anticorps anti-ICAM est un anticorps anti-ICAM-1.

8. Composition selon la revendication 1, dans laquelle ledit agent qui interrompt ou perturbe ladite interaction entre CD40 et son ligand est un anticorps anti-CD40.

9. Composition selon la revendication 1, dans laquelle ledit agent qui interrompt ou perturbe ladite interaction entre CD40 et son ligand est un anticorps anti-CD40 ligand ou un fragment actif de celui-ci.

10. Composition selon l'une quelconque des revendications 1 à 9, dans laquelle ledit agent qui interrompt ou perturbe ladite interaction ICAM-LFA-1 et/ou ledit agent qui interrompt ou perturbe ladite interaction entre CD40 et son ligand est un agent non-anticorps.

11. Utilisation d'une composition pharmaceutique selon l'une quelconque des revendications 1 à 10 dans la préparation d'un médicament.

12. Utilisation d'un rapport synergique de (i) un anticorps qui interrompt ou perturbe l'interaction ICAM-LFA-1 ou un fragment actif de celui-ci et (ii) un anticorps qui interrompt ou perturbe l'interaction entre CD40 et son ligand ou un fragment actif de celui-ci dans la préparation d'un médicament destiné à être utilisé dans le traitement de la polyarthrite rhumatoïde ou du lupus érythémateux disséminé.

13. Utilisation selon la revendication 12, dans laquelle ladite ICAM est ICAM-1.

14. Utilisation selon la revendication 12, dans laquelle ledit anticorps qui interrompt ou perturbe ladite interaction ICAM-LFA-1 est un anticorps anti-ICAM et dans laquelle ledit anticorps qui interrompt ou perturbe ladite interaction entre CD40 et son ligand est un anticorps anti-CD40 ligand.

15. Utilisation selon la revendication 12, dans laquelle ledit anticorps anti-ICAM est un anticorps anti-ICAM-1.

16. Utilisation selon la revendication 12, dans laquelle ledit anticorps qui interrompt ou perturbe ladite interaction ICAM-LFA-1 est un anticorps anti-LFA-1 ou un fragment actif de celui-ci, et dans laquelle ledit anticorps qui interrompt ou perturbe ladite interaction entre CD40 et son ligand est un anticorps anti-CD40 ligand ou un fragment actif de celui-ci.

17. Utilisation selon l'une quelconque des revendications 12 à 16, dans laquelle le lupus érythémateux disséminé est en phase terminale.

18. Utilisation selon l'une quelconque des revendications 12 à 17, dans laquelle ledit médicament comprend en outre des agents autres que (i) ledit agent qui interrompt ou perturbe l'interaction ICM-LFA-1 et (ii) ledit agent qui interrompt ou perturbe l'interaction entre CD40 et son ligand.

19. Utilisation d'un rapport synergique de (i) un agent qui interrompt ou perturbe l'interaction ICAM-LFA-1 et (ii) un agent qui interrompt ou perturbe l'interaction entre CD40 et son ligand dans la préparation d'un médicament destiné à être utilisé dans le traitement d'une maladie auto-immune.

20. Utilisation selon la revendication 19, dans laquelle la maladie auto-immune est la polyarthrite rhumatoïde ou le lupus érythémateux disséminé.

21. Produit comprenant un rapport synergique de (i) un agent qui interrompt ou perturbe l'interaction ICAM-LFA-1 et (ii) un agent qui interrompt ou perturbe l'interaction entre CD40 et son ligand, se présentant sous la forme d'une préparation combinée pour une utilisation simultanée, séquentielle ou séparée dans le traitement des maladies auto-immunes.
